# EUROPEAN PATENT SPECIFICATION

(11) **EP 3 831 352 B1**
(45) Date of publication and mention of the grant of the patent: **24.07.2024**
(21) Application number: 19844355.8
(22) Date of filing: 03.07.2019
(51) Int. Cl.: A61F 13/49, A61F 13/494, A61F 13/496

(54) **DISPOSABLE WEARABLE ARTICLE**
WEARABLE-EINWEGARTIKEL
ARTICLE JETABLE POUVANT ÊTRE PORTÉ

(30) Priority: 31.07.2018 JP 2018143835; 30.05.2019 JP 2019101719
(43) Date of publication of application: 09.06.2021
(73) Proprietor: Daio Paper Corporation, Ehime 799-0492 (JP)
(72) Inventor: YAMAMOTO, Shohei, Shikokuchuo-shi, Ehime 799-0431 (JP)
(74) Representative: Williams Powell
(86) International application number: PCT/JP2019/026438
(87) International publication number: WO 2020/026684

(56) References cited:
- WO-A1-2017/212857
- JP-A- 2011 110 317
- JP-A- 2012 161 557
- JP-A- 2014 117 349
- JP-A- H11 188 057
- US-A- 5 275 590
- US-A1- 2011 196 327
- US-A1- 2014 163 500
- US-B1- 6 383 170

## Description

### Technical Field

The present invention relates to a disposable wearable article, in which the touch feeling (flexibility, cushioning properties) of the side portions of a crotch portion is improved.

### Background Art

One of the typical examples of disposable wearable articles is an underpants-type disposable diaper. The underpants-type disposable diaper includes an outer body that constitutes at least a lower torso portion of a front body and a back body, an inner body including an absorber, which is attached to the outer body so as to extend from the front body to the back body, and by bonding both side portions of the outer body of the front body and both side portions of the outer body of the back body to form a side seal portion, a waist opening, and a pair of left and right leg openings are generally formed (refer to, for example, Patent Literature 1).

In the conventional underpants-type disposable diaper, the side portions of the crotch portion are formed only by the outer body, but recently, the side portion of the crotch portion is often formed by the side portion of the inner body (for example, refer to Patent Literature 1).

A tape-type disposable diaper (refer to Patent Literatures 2 to 4) is also a typical example of disposable wearable articles. The conventional tape-type disposable diaper includes a crotch portion including the center in the front-back direction, a ventral part extending from the center in the front-back direction to the front side, and a dorsal part extending backward from the center in the front-back direction. The ventral part and the dorsal part include a main unit section located between the left and right wing parts. At least the dorsal part includes a wing part extending to the both left and right sides in the width direction from the crotch portion, and the wing part includes a connecting portion that is detachably connected to the outer surface of the ventral part. In use, the wing parts are turned from both sides of the waist to the outer surface of the ventral part to connect the connecting portions of the wing parts to the outer surface of the ventral part.

Conventionally, as a tape-type disposable diaper, a separate-type is known in which separately manufactured connecting tapes are attached to side edges of the assembly forming the main unit section, and these connecting tapes become the wing parts (refer to, for example, Patent Literature 3 and 4), in addition to a one unit-type in which side flaps extending laterally from the side edges of an absorber are cut around the leg portions, and uncut portions are left as wing parts (refer to, for example, Patent Literatures 2). Further, as a separate type, it is also proposed to attach a unit including left and right wing parts to the assembly forming the main unit section (refer to, for example, Patent Literature 6).

In such a tape-type disposable diaper, the side portion of the crotch portion is formed by the side portion of the side flap.

Now that, in these disposable wearable articles, it is known that, in order to secure fitting to the legs, between the sheet layers on the side portion of the crotch portion, an elongated elastic member is built in along the direction along the leg portions or along the front-back direction.

However, in a conventional disposable wearable article, when the product is held or worn by hand during wearing or purchasing, there is a room for improvement in the touch feeling of the side portions of the crotch portion, particularly the flexibility and cushioning property (compression resilience) of sheet layers on the side portions of the crotch portion.

### Citation List

### Patent Literature

Patent Literature 1: JP 2006-122396 A
Patent Literature 2: JP 2016-083077 A
Patent Literature 3: JP 2010-22550 A
Patent Literature 4: JP 2011-72736 A
US 5275590 A discloses a disposable absorbent garment, illustrated as a disposable diaper, which includes a backsheet, an absorbent panel positioned on top of the backsheet, and a composite, three-component topsheet assembly positioned on top of the absorbent panel.
US 6383170 B1 discloses a disposable diaper including a pair of elasticized gasket cuffs disposed in transversely opposite sides of the diaper, a pair of elasticized barrier cuffs disposed adjacent the gasket cuffs and a three-dimensional feces receiving section extending at least partially across a crotch region of the diaper.
US 2011/196327 A1 discloses an absorbent article to be worn about the lower torso. The absorbent article comprises a chassis comprising a topsheet, a backsheet, an absorbent core disposed between the topsheet and the backsheet, and a pair of longitudinal barrier cuffs attached to the chassis.
US 2014/163500 A1 discloses an absorbent article that comprises an absorbent core disposed at least partially intermediate a topsheet and a backsheet and a core wrap enclosing an absorbent material.
WO 2017/212857 A1 (in the name of the present applicant) discloses a two-part exterior shell-type pants-type disposable diaper in which a cover nonwoven fabric that covers the rear surface of an inner layer and extends from between the front-side outer shell and the inner layer to between the rear-side outer shell and the inner layer, the cover nonwoven fabric extends at least from the rear side of the base edge of one side gather to the rear side of the base edge of the other side gather, the cover nonwoven fabric being provided with a large number of front-rear through-holes with intervals therebetween, from between the outer shell and the inner layer to between the rear-side outer shell and inner layer, and the side edge of the cover nonwoven fabric being positioned at the same position as or further to the center in the width direction than the side edge of the narrowest part of the absorbent.

### Summary of Invention

### Technical Problem

Therefore, a main object of the present invention is to improve the touch feeling of the side portions of the crotch portion.

### Solution to Problem

### <First Aspect>

The disposable wearable article which has solved the above-described problem is as defined in claim 1.

### (Function and Effect)

In the present disposable wearable article, the side elastic member contracts, whereby the portion including the hollow portion along the side flap contracts in the front-back direction, and pleats of the side gathers are formed side by side in the front-back direction. Here, when the side gathers are formed in the portion including the hollow portion of the side flap, the pleats of the side gathers are formed by the hollow portion being greatly expanded, and the pleats are large and soft (that is, rich in cushioning properties). Therefore, due to the large and soft pleats, the touch feeling of the side portion of the crotch portion is improved (becomes soft and fluffy).

In this structure, the pleats are independently formed in the portion located between a plurality of the hollow portions, and the pleats are overlapped with each other such that the pleats having a better cushioning property are formed on the side portions of the side flaps.

When the hollow portion is formed by the folded-back sheet layer, it is preferable because the cushioning property of the pleats formed in the hollow portion is particularly improved by the restoring force against folding back.

In this structure, by disposing the double folded-back sheet layers on the side flap, the hollow portion can be formed in three tiers (three chambers in the thickness direction) at least on the side portions of the side flap. In this case, since each of the double folded-back sheet layers has a restoring force against folding back, a pleat having a superior cushioning property is formed on the side portion of the side flap with less sheet material.

### <Second Aspect>

In the disposable wearable article according to the first aspect,
the hollow portion extends from the outside edge of the side flap to a position separated from the outside edge by 2 mm or more toward the center of the disposable wearable articlein the width direction.

### (Function and Effect)

The position and width of the hollow portion may be appropriately determined, but in the normal case, it is preferably within the range of the present aspect.

### <Third Aspect>

In the disposable wearable article according to any one of the first or second aspects,
a portion in the side flap of 2 to 15 mm from the outside edge toward the center of the disposable wearable article in the width direction does not include a side elastic member, and includes a part or all of the hollow portion.

### (Function and Effect)

As described above, the side elastic member is sufficiently separated from the side edge of the side flap, and if the separated part includes a part or all of the hollow portion, a large pleat that is rich in flexibility and cushioning is formed in the entire thickness direction on the side of the portion that is pressed against the skin (that is, the part with the side elastic member) when the product is held by hand for wearing or purchasing, or during wearing, and it is particularly preferable in improving the touch feeling of the ends of leg openings.

### <Fourth Aspect>

In the disposable wearable article according to any one of the first to third aspects,
at least one side elastic member is provided on the front surface side of the hollow portion.

### (Function and Effect)

The positional relationship between the hollow portion and the side elastic member can be appropriately determined, but when at least one side elastic member is provided on the front surface side of the hollow portion, since the contraction force of the side elastic member is directly applied to the hollow portion, the shape maintaining property of the pleats in the hollow portion is preferably improved.

### <Fifth Aspect>

In the disposable wearable article according to any of the first to fourth aspects,
rising gathers rise up from a position on a center side in the width direction relative to the one or more side elastic members and extend along the front-back direction,
each rising gather includes a gather sheet that is folded in two at a tip portion to form a two-layered structure, and an elongated gather elastic member is provided along at least the interlayer of the two-layered structure in the front-back direction,
both side portions of each side flap have a first part in which the two-layered structure made of the gather sheet extends from a starting point to the outside edge of the side flap and a second part in which the two-layered structure made of the gather sheet is folded back at the outside edge of the side flap and extends toward the center of the disposable wearable article in the width direction, and
the double folded-back sheet layer is the first part and the second part of the gather sheet.

### (Function and Effect)

In a disposable wearable article, it is common to provide a rising gather as in the present aspect. That is, a general rising gather has a gather sheet having a two-layered structure that is folded in two at the tip portion. Therefore, it is preferable to provide the above-described hollow portion by extending the two-layered structure of this gather sheet and folding it back at the side edge. As a result, a pleat having a superior cushioning property is formed on the side portion of the side flap with less sheet material.

### <Sixth Aspect>

In the disposable wearable article according to any one of the first to fifth aspects,
multiple side elastic members are provided at intervals in the width direction, and
a stretch rate of the side elastic member in the side stretchable region in an unfolded state is lower as it is located laterally.

### (Function and Effect)

By providing multiple side elastic members having different stretch rates in this way, the rise of the side stretchable region to the skin side is loose, and the side stretchable region is likely to have an appropriate wearing state. If the side stretchable region rises tightly, the side flap may be folded back inward and sandwiched between the leg and the surface of the disposable wearable article, and it may lead to leakage.

### <Seventh Aspect>

In the disposable wearable article according to any one of the first to sixth aspects,
an underpants-type disposable wearable article includes an outer body that forms at least a lower torso portion of a front body and a back body, an inner body that incorporates an absorber and is attached to the outer body so as to extend from the front body to the back body, a waist opening, and a pair of left and right leg openings, and
the side flaps are provided on both side portions of the inner body.

### (Function and Effect)

When the present disposable wearable article is applied to underpants-type disposable wearable articles, it is desirable to provide the side flaps having no absorber on both side portions of the inner body, and to provide hollow portions in the side flaps.

### Advantageous Effects of Invention

According to the present invention, the touch feeling of the side portions of the crotch portion improves, and other advantages are brought about.

### Brief Description of Drawings

Fig. 1 is a plan view illustrating the inner surface of an underpants-type disposable diaper in an unfolded state.
Fig. 2 is a plan view illustrating the outer surface of an underpants-type disposable diaper in an unfolded state.
Fig. 3 is a cross-sectional view taken along line 2-2 in Fig. 1.
Fig. 4 is a cross-sectional view taken along line 3-3 in Fig. 1.
Fig. 5(a) is a cross-sectional view taken along line 4-4 in Fig. 1. Fig. 5(b) is a cross-sectional view taken along line 5-5 in Fig. 1.
Fig. 6 is a perspective view of an underpants-type disposable diaper.
Fig. 7 is a plan view illustrating the outer surface of the inner body in an unfolded state together with the outline of an outer body.
Fig. 8 is a plan view illustrating the outer surface of the inner body in an unfolded state together with the outline of an outer body.
Fig. 9 is a cross-sectional view of another example corresponding to a cross-section taken along line 2-2 in Fig. 1.
Fig. 10 is a cross-sectional view of another example corresponding to a cross-section taken along line 3-3 in Fig. 1.
Fig. 11 is an enlarged cross-sectional view of a main part of Fig. 3.
Fig. 12 is an enlarged cross-sectional view of a main part of Fig. 9.
Fig. 13(a) is a cross-sectional view taken along line 4-4 in Fig. 1. Fig. 13(b) is a cross-sectional view taken along line 5-5 in Fig. 1.
Fig. 14 is a perspective view of an underpants-type disposable diaper.
Fig. 15 is a cross-sectional view of a front outer body and a back outer body.
Fig. 16 is a cross-sectional view of a front outer body and a back outer body.
Fig. 17 is a cross-sectional view of a front outer body and a back outer body.
Fig. 18 is a cross-sectional view of a front outer body and a back outer body.
Fig. 19 is a cross-sectional view of another example corresponding to a cross section taken along line 4-4 in Fig. 1.

### Description of Embodiments

Hereinafter, as an example of an underpants-type disposable wearable article, an underpants-type disposable diaper will be described in detail with reference to the accompanying drawings. The dotted pattern portion in the cross sectional view indicates an adhesive as a bonding means for bonding respective constituent members positioned on the front surface side and the back surface side thereof, and the solid, bead, curtain, summit, or spiral application, or pattern coating (transfer of a hot melt adhesive in a letterpress method) of the hot melt adhesive, or the fixed portion of the elastic member is formed by application on the outer peripheral surface of the elastic member, such as a comb gun or a surewrap application in place of or in addition to the above. Examples of the hot melt adhesive include, but are not limited to, adhesives of an EVA-based, pressure sensitive adhesion rubber-based (elastomer-based), polyolefin-based, and polyester/polyamide-based. As a bonding means for bonding respective constituent members, a means by material welding such as heat sealing or ultrasonic sealing can also be used.

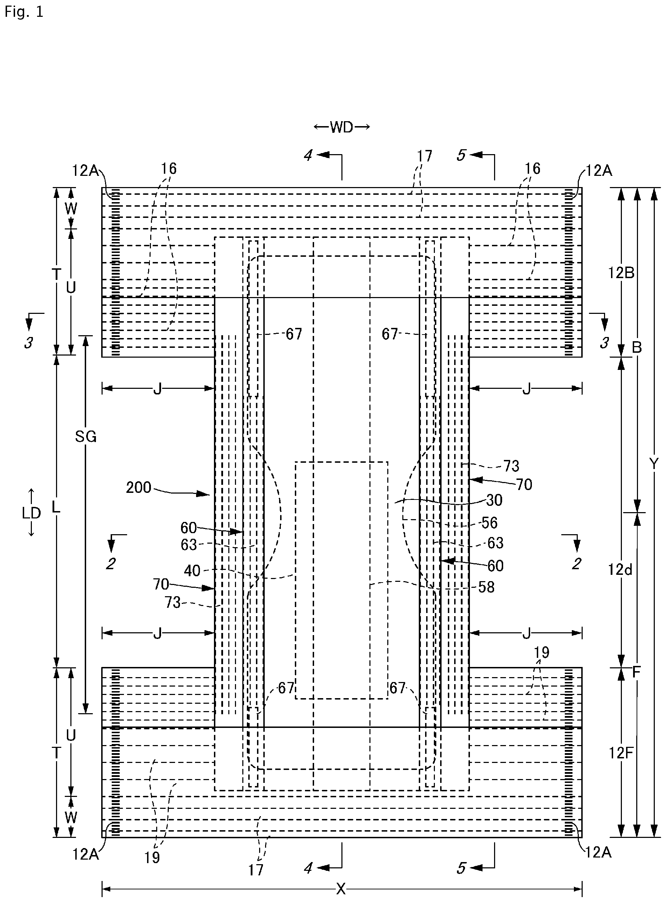

Figs. 1 to 6 illustrate an example of an underpants-type disposable diaper. The present underpants-type disposable diaper is provided with a rectangular front outer body 12F that forms at least a lower torso portion of the front body F, a rectangular back outer body 12B that forms at least the lower torso portion of the back body B, and the inner body 200 provided inside the outer bodies 12F and 12B so as to extend from the front outer body 12F through the crotch portion to the back outer body 12B. Both side portions of the front outer body 12F and both side portions of the back outer body 12B are bonded to form a side seal portion 12A, whereby openings formed by the front and back end portions of the outer bodies 12F and 12B are a waist opening WO through which the trunk of the wearer passes, and the portions surrounded by the lower edges of the outer bodies 12F and 12B and the side edges of the inner body 200 on both sides in the width direction of the inner body 200 are leg openings LO through which the legs pass. The inner body 200 is a portion to absorb and hold excrement such as urine, and the outer bodies 12F and 12B are portions for supporting the inner body 200 with respect to the wearer's body. The reference character Y denotes the maximum length of a diaper in an unfolded state (the length in the front-back direction from the edge of the waist opening WO of the front body F to the edge of the waist opening WO of the back body B), and the reference character X denotes the maximum width of a diaper in an unfolded state.

The present underpants-type disposable diaper has a lower torso region T defined as a front-back direction range (a range in the front-back direction from the waist opening WO to the upper end of the leg opening LO) having the side seal portion 12A and an intermediate region L defined as a front-back direction range of a portion forming the leg opening LO (between the region in the front-back direction having the side seal portion 12A of the front body F and the region in the front-back direction having the side seal portion 12A of the back body B). The lower torso region T can be divided into a "waist portion" W which conceptually forms an edge portion of the waist opening and an "under-waist portion" U which is a portion lower than the waist portion W. Normally, when there is a boundary where stretching stress of the width direction WD changes in the lower torso region T (for example, the fineness and stretch rate of the elastic member change), the waist opening WO side closest to the boundary on the waist opening WO side is the waist portion W. When there is no such boundary, the waist extended section 12E extending toward the waist opening WO side from the absorber 56 or the inner body 200 is the waist portion W. The length in the front-back direction varies depending on the size of a product and can be appropriately determined. For example, the waist portion W can be set to 15 to 40 mm, and the under-waist portion U can be set to 65 to 120 mm. On the other hand, both side edges of the intermediate region L are constricted in a U shape or a curved shape along the periphery of the legs of a wearer, and this is a site for inserting the wearer's legs. As a result, the underpants-type disposable diaper in an unfolded state has a substantially hourglass shape as a whole.

### (Inner Body)

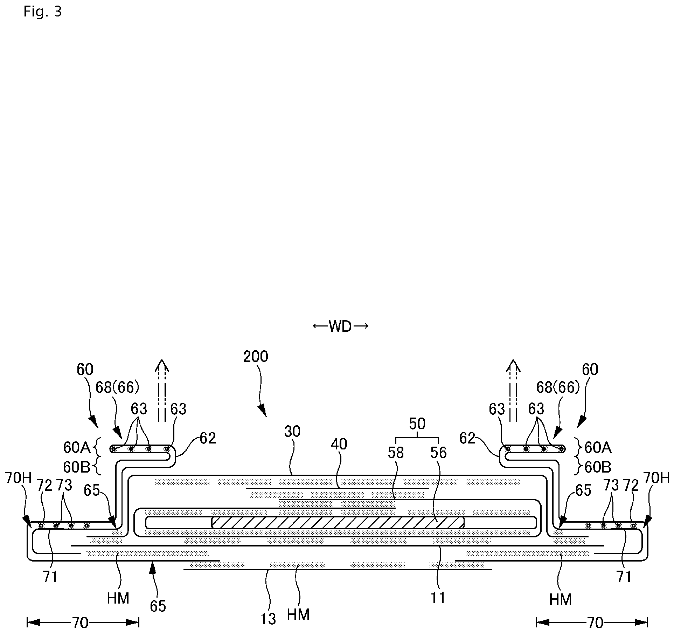

The inner body 200 can have an arbitrary shape, but in the illustrated example, it is rectangular. As illustrated in Figs. 3 to 5, the inner body 200 is provided with a top sheet 30 which is positioned on the body side, a liquid impervious sheet 11, and an absorbent element 50 interposed therebetween, and is a main unit section that plays a role of an absorbent function. The reference character 40 denotes an intermediate sheet provided between the top sheet 30 and the absorbent element 50 in order to promptly transfer liquid having permeated through the top sheet 30 to the absorbent element 50. The reference character 60 denotes the rising gather 60 extending from both side portions of the inner body 200 so as to be in contact with a leg portion of a wearer in order to prevent excrement from leaking to both sides of the inner body 200.

### (Top Sheet)

The top sheet 30 has a property of permeating liquid, and examples of the top sheet 30 include a perforated or imperforate nonwoven fabric and a porous plastic sheet. Further, the top sheet 30 may be made of one sheet or a laminated sheet obtained by bonding two or more sheets. Similarly, the top sheet 30 may be composed of one sheet or two or more sheets with respect to the plane direction.

Both side portions of the top sheet 30 may be folded back to the back surface side at the side edge of the absorbent element 50 or protruded laterally beyond the side edge of the absorbent element 50 without folding back.

For the purpose of preventing positional deviation with respect to the member on the back surface side, it is desirable that the top sheet 30 be fixed to a member adjacent to the back surface side by a bonding means by material welding such as heat sealing or ultrasonic sealing or a hot melt adhesive. In the illustrated example, the top sheet 30 is fixed to the surface of the intermediate sheet 40 and the surface of the package sheet 58, which is located on the front surface side of the absorber 56, by a hot melt adhesive applied on the back surface thereof.

### (Intermediate Sheet)

To quickly transfer liquid having permeated through the top sheet 30 to the absorber, it is possible to provide an intermediate sheet (also referred to as a "second sheet") 40 having a higher liquid permeation rate than the top sheet 30. This intermediate sheet 40 is intended to quickly transfer the liquid to the absorber 56 to improve the absorption performance of the absorber 56 and also prevent the returning phenomenon of the absorbed liquid from the absorber 56. The intermediate sheet 40 can also be omitted.

Examples of the intermediate sheet 40 include the same material as the top sheet 30, a spun lace nonwoven fabric, a spun bond nonwoven fabric, an SMS nonwoven fabric, a pulp nonwoven fabric, a mixed sheet of pulp and rayon, a point bond nonwoven fabric, or a crepe paper. In particular, an air-through nonwoven fabric is preferable because it is bulky. It is preferable to use a composite fiber having a core-sheath structure for the air-through nonwoven fabric. In this case, resin used for the core may be polypropylene (PP), but polyester (PET) having high rigidity is preferable. The basis weight is preferably 17 to 80 g/m², more preferably 25 to 60 g/m². The fineness of the raw material fiber of the nonwoven fabric is preferably 2.0 to 10 dtex. To increase the bulkiness of the nonwoven fabric, it is also preferable to use eccentric fibers whose core is not in the center, hollow fibers, eccentric and hollow fibers, as mixed fibers of all or a part of the raw material fibers.

Although the intermediate sheet 40 in the illustrated example is shorter than the width of the absorber 56 and disposed at the center, it may be provided throughout the maximum width. The length of the intermediate sheet 40 in the front-back direction may be the same as the maximum length of a diaper, may be the same as the length of the absorbent element 50, or may be within a short length range around a region receiving a liquid.

For the purpose of preventing positional deviation with respect to the member on the back surface side, it is desirable that the intermediate sheet 40 be fixed to a member adjacent to the back surface side by a bonding means by material welding such as heat sealing or ultrasonic sealing or a hot melt adhesive. In the illustrated example, the intermediate sheet 40 is fixed to the surface of the package sheet 58, which is located on the front surface side of the absorber 56, by a hot melt adhesive applied on the back surface thereof.

### (Liquid Impervious Sheet)

The material of the liquid impervious sheet 11 is not particularly limited, but examples of the material include a plastic film made of a polyolefin-based resin such as polyethylene and polypropylene, a laminated nonwoven fabric having a plastic film on the surface of a nonwoven fabric, and a laminated sheet obtained by bonding nonwoven fabrics or the like on a plastic film. In the liquid impervious sheet 11, it is preferable to use a material having liquid impermeability and moisture penetrability that has been favorably used from the viewpoint of prevention of stuffiness. As the moisture-penetrable plastic film, a microporous plastic film is widely used. The microporous plastic film is obtained by kneading an inorganic filler in a polyolefin-based resin such as polyethylene or polypropylene, molding the resin into a sheet, and thereafter stretching the sheet in a monoaxial or biaxial direction . In addition to this, a nonwoven fabric using a micro-denier fiber and a liquid impervious sheet in which a plastic film is not used by increasing leakage resistance by reducing gaps of fiber by applying heat and pressure and by coating with super absorbent polymer, a hydrophobic resin, or a water repellent agent can also be used as the liquid impervious sheet 11. However, it is desirable to use a resin film to obtain sufficient adhesive strength in the case of adhering to the cover nonwoven fabric 13 to be described later via a hot melt adhesive.

To enhance leakage resistance, the liquid impervious sheet 11 may have a width that fits on the back surface side of the absorbent element 50 as illustrated in the drawing and can also be disposed around the both sides of the absorbent element 50 to extend to the both side portions of the side surface of the top sheet 30 of the absorbent element 50. It is appropriate that the width of this extending portion is about 5 to 20 mm on each side.

### (Absorbent Element)

The absorbent element 50 has the absorber 56 and a package sheet 58 packaging the entire absorber 56. The package sheet 58 can also be omitted.

### (Absorber)

The absorber 56 can be formed of an assembly of fibers. As this fiber assembly, besides those obtained by accumulating short fibers such as fluff pulp and synthetic fibers, a filament assembly obtained by opening tows (fiber bundles) of synthetic fibers such as cellulose acetate as required can also be used. When fluff pulp or short fibers are accumulated, fiber basis weight can be set to, for example, about 100 to 300 g/m², and in the case of a filament assembly, fiber basis weight can be set to about 30 to 120 g/m². In the case of a synthetic fiber, the fineness is, for example, 1 to 16 dtex, preferably 1 to 10 dtex, more preferably 1 to 5 dtex. In the case of filament assembly, the filaments may be non-crimped fibers, but are preferably crimped fibers. The degree of crimp of the crimped fiber can be, for example, about 5 to 75 crimps, preferably about 10 to 50 crimps, and more preferably about 15 to 50 crimps per 2.54 cm. In addition, crimped fibers which are uniformly crimped can be used. It is preferable to disperse and hold the super absorbent polymer particles in the absorber 56.

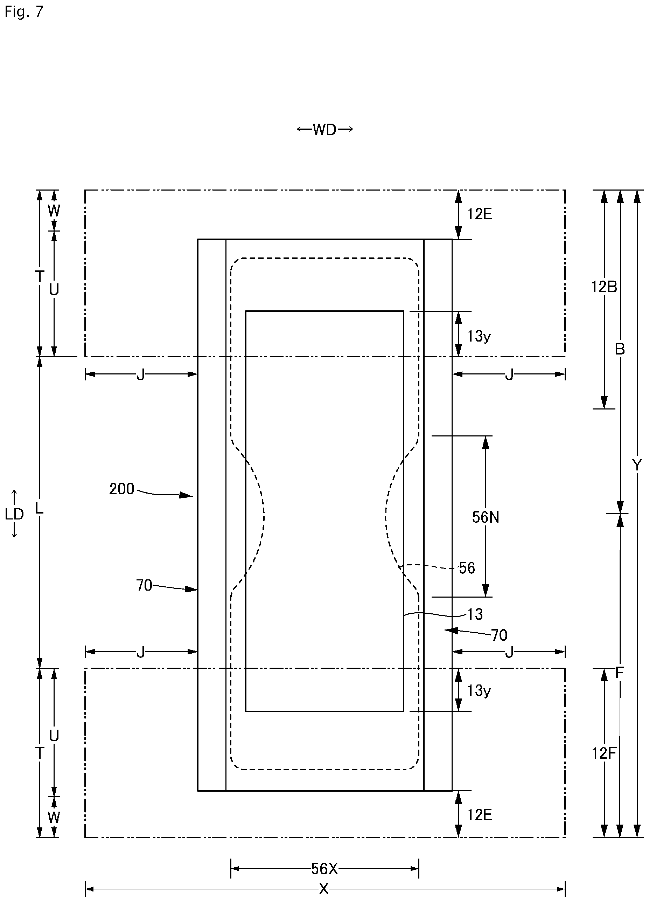

The absorber 56 may have a rectangular shape, and, as illustrated in Fig. 7, etc., preferably has an hourglass shape having a narrowing portion 56N having a width narrower than both front and back sides in the middle in the front-back direction, since the fitting of the absorber 56 itself and the rising gather 60 around the legs is improved.

The dimension of the absorber 56 can be appropriately determined as far as it extends over the front, back, right and left of the position of a ureteral outlet, but it is preferable that it extends to the peripheral portion of the inner body 200 or the vicinity thereof in the front-back direction LD and the width direction WD. The reference character 56X denotes the maximum width of the absorber 56.

### (Super Absorbent Polymer Particle)

The absorber 56 can contain super absorbent polymer particles partially or entirely. The super absorbent polymer particle means "powder" in addition to "particle". The super absorbent polymer particles 54 used for this type of disposable diapers can be used as they are, and it is desirable that the proportion of particles remaining on a sieve is 30% by weight or less by sieving (shaking for 5 minutes) using, for example, a standard sieve of 500 um (JIS Z 8801-1: 2006). In addition, it is desirable that the proportion of particles remaining on the sieve by sieving (shaking for 5 minutes) using a standard sieve of 180 um (JIS Z 8801-1: 2006) be 60% by weight or more.

The material of the super absorbent polymer particles is not particularly limited, but materials having a water absorption capacity of 40 g/g or more are suitable. Examples of the super absorbent polymer particles include starch-based, cellulose-based, and synthetic polymer-based particles, and starch-acrylic acid (salt) graft copolymers, saponified starch-acrylonitrile copolymers, crosslinked sodium carboxymethylcellulose, and acrylic acid (salt) polymers can be used. As the shape of the super absorbent polymer particles, particulate materials which are usually used are preferable, but other shapes can also be used.

The super absorbent polymer particles having a water absorption rate of 70 seconds or less, particularly 40 seconds or less, are suitably used. If the water absorption speed is too slow, back-flow, in which the liquid fed into the absorber 56 returns to the outside of the absorber 56, is likely to occur.

As the super absorbent polymer particles, those having a gel strength of 1,000 Pa or more are preferably used. Thereby, even when the absorber 56 is bulky, it is possible to effectively suppress stickiness after liquid absorption.

The basis weight of the super absorbent polymer particles can be appropriately determined according to the absorption amount required for the use of the absorber 56. Therefore, although it cannot be said unconditionally, the basis weight can be 50 to 350 g/m². When the basis weight of the polymer is less than 50 g/m², it is difficult to ensure the absorption amount. When it exceeds 350 g/m², the effect is saturated.

### (Package Sheet)

When the package sheet 58 is used, tissue paper, particularly crepe paper, a nonwoven fabric, a poly lamina nonwoven fabric, a sheet with small openings can be used as the material. However, it is desirable that the sheet from which the super absorbent polymer particles do not come off is used. When a nonwoven fabric is used in place of crepe paper, a hydrophilic SMS nonwoven fabric (SMS, SSMMS, etc.) is particularly suitable, and polypropylene, polyethylene/polypropylene composite material and the like can be used as the material. The basis weight is desirably 5 to 40 g/m², particularly desirably 10 to 30 g/m².

The wrapping structure of the package sheet 58 can be appropriately determined. However, from the viewpoints of ease of manufacturing and prevention of leakage of super absorbent polymer particles from the front and back end edges, it is preferable that the package sheet 58 is wound around in a cylindrical shape so as to surround the front and back surfaces and both side surfaces of the absorber 56, the front and back edge portions are protruded from the front and back of the absorber 56, and the lap winding portion and the overlapping portion of the front-back protruding portions are bonded by bonding means such as hot melt adhesive, material welding, or the like.

### (Rising Gather)

The rising gather 60 has a rising portion 68 that rises from the side portion of the inner body 200, and the rising portion 68 is in contact with the range from the wearer's groin region to the gluteal region through the leg portion to prevent side leakage. In the rising gathers 60 in the illustrated example, the root-side portion 60B stands diagonally toward the center in the width direction, and the portion 60A closer to the tip side from the intermediate portion stands diagonally outward in the width direction, but this is not limited thereto, and it can be appropriately changed to a form of standing on the center side in the width direction as a whole or the like.

To be more specific, the rising gather 60 in the illustrated example is formed by folding back in two, in the width direction WD at a portion to be a tip portion, a belt shaped gather sheet 62 having a length equal to the length in the front-back direction of the inner body 200, and fixing a plurality of elongated gather elastic members 63 between the folded-back portion and an adjacent sheet with intervals in the width direction WD in a stretched state along the longitudinal direction. A base portion (an end portion on the side opposite to the sheet folded-back portion in the width direction WD) positioned on the opposite side of the tip portion of the rising gathers 60 is the root portion 65 fixed to the side portion of the inner body 200, and portions other than the root portion 65 are a main unit section 66 extending from the root portion 65 (a portion on the folded-back portion side). Further, the main unit section 66 has a root-side portion 60B extending toward the center side in the width direction and a tip-side portion 60A folded back at the tip of the root-side portion 60B and extending outward in the width direction. Both end portions of the main unit section 66 in the front-back direction are set to be fallen portions 67 fixed to the side surface of the top sheet 30 in a fallen state, while the intermediate portion in the front-back direction positioned therebetween is a non-fixed rising portion 68, and the gather elastic member 63 along the front-back direction LD is fixed to at least the tip portion of the rising portion 68 in a stretched state.

In the rising gather 60 formed as described above, due to the contraction force of the gather elastic member 63, the rising portion 68 rises so as to contact the skin as indicated by the arrow in Fig. 3.

As with the rising gather 60 in the illustrated example, in the bending structure in which the main unit section 66 is composed of the root-side portion 60B extending to the center side in the width direction and the tip-side portion 60A that is folded back at the tip of the root-side portion 60B and extends outward in the width direction, and at the fallen portion 67, the tip-side portion 60A and the root-side portion 60B are bonded in a fallen state, and the root-side portion 60B is bonded to the top sheet 30 in a fallen state. At least one of a hot melt adhesive by various application methods and material welding such as heat sealing or ultrasonic sealing can be used to bond the opposing surfaces of the fallen portion 67. In this case, the bonding of the root-side portion 60B and the top sheet 30 and the bonding of the tip-side portion 60A and the root-side portion 60B may be performed by the same means or different means. For example, it is preferable that the root-side portion 60B and the top sheet 30 are bonded by a hot melt adhesive, and the tip-side portion 60A and the root-side portion 60B are bonded by material welding.

As the gather sheet 62, a nonwoven fabric which is flexible and excellent in uniformity and concealing property such as a spun bond nonwoven fabric (SS, SSS, etc.), SMS nonwoven fabric (SMS, SSMMS, etc.), meltblown nonwoven fabric, and on which a water repellent process is performed by silicone as necessary, can be preferably used. In this case, the fiber basis weight of the nonwoven fabric is preferably about 10 to 30 g/m². As the gather elastic member 63, a rubber thread or the like can be used. When a spandex rubber thread is used, the fineness is preferably 470 to 1240 dtex, more preferably 620 to 940 dtex. The stretch rate of the gather elastic member 63 in the attached state is preferably 150 to 350%, more preferably 200 to 300%. The number of gather elastic members 63 is preferably 2 to 6, and more preferably 3 to 5. An appropriate arrangement interval 60d of the gather elastic members 63 is 3 to 10 mm. With such a configuration, a range in which the gather elastic member 63 is disposed easily comes into surface contact with the skin. The gather elastic member 63 may be disposed not only on the tip side but also on the root side.

In the rising portion 68 of the rising gathers 60, at least one of hot melt adhesives by various application methods and a fixing means by material welding such as a heat sealing, ultrasonic sealing, and the like can be used for bonding the inner layer and the outer layer of the gather sheet 62 and fixing the gather elastic member 63 sandwiched therebetween. When the inner layer and the outer layer of the gather sheet 62 are entirely bonded together, the flexibility is impaired. Therefore, it is preferable that the portion other than the adhesive portion of the gather elastic member 63 is not adhered or is weakly adhered. In the illustrated example, by applying a hot melt adhesive only to the outer peripheral surface of the gather elastic member 63 by an application means such as a comb gun or a surewrap nozzle and sandwiching it between the inner layer and the outer layer of the gather sheet 62, fixing of the gather elastic member 63 to the inner layer and the outer layer of the gather sheet 62 and fixing between the inner layer and the outer layer of the gather sheet 62 are carried out by using only the hot melt adhesive applied to the outer peripheral surface of the gather elastic member 63.

Similarly, at least one of a hot melt adhesive by various application methods and material welding such as heat sealing or ultrasonic sealing can be used to fix the fallen portion 67.

### (Side Flap Portion)

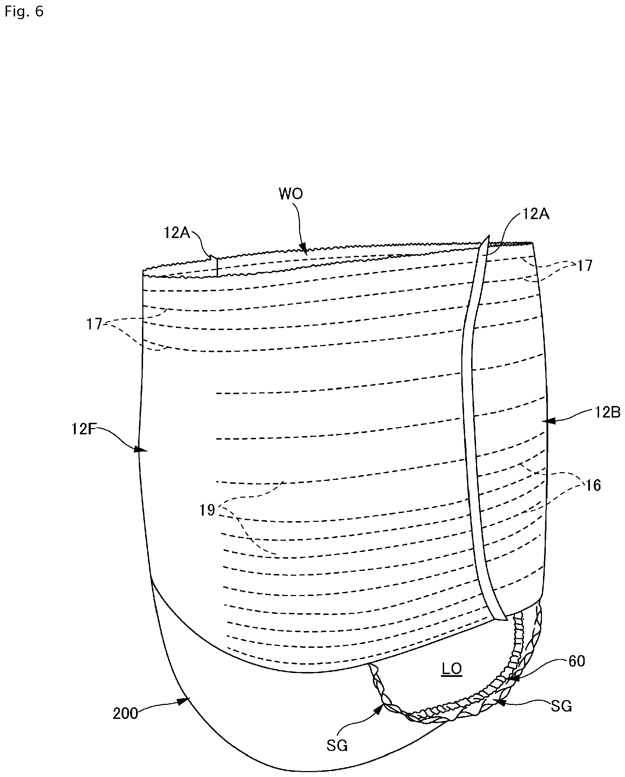

On both side portions of the inner body 200, the side flaps 70 extending laterally of the absorber 56 are provided, and each side flap 70 preferably has a hollow portion 70H extending along the side edge thereof in the front-back direction LD. Further, each side flap 70 has a side stretchable region SG in which one or a plurality of elongated side elastic members 73 spaced from each other is provided along the front-back direction LD. As a result, due to the contraction of the side elastic member 73, a portion of the side flap 70 including the hollow portion 70H contracts in the front-back direction LD, and a side gather in which a large number of pleats are arranged in the front-back direction LD is formed. Here, when the side gathers are formed in the portion of the side flap 70 including the hollow portion 70H, as illustrated in Fig. 6, the pleats of the side gathers are formed by the hollow portion 70H being greatly expanded, and are large and soft (that is, rich in cushioning properties). Therefore, due to the large and soft pleats, the touch feeling of the side portion of the crotch portion is improved (becomes soft and fluffy).

It is preferable that the side flap 70 have a first sheet layer 71 facing the back surface side of the side elastic member 73 and a second sheet layer 72 facing the front surface side of the side elastic member 73 for fixing the side elastic member 73.

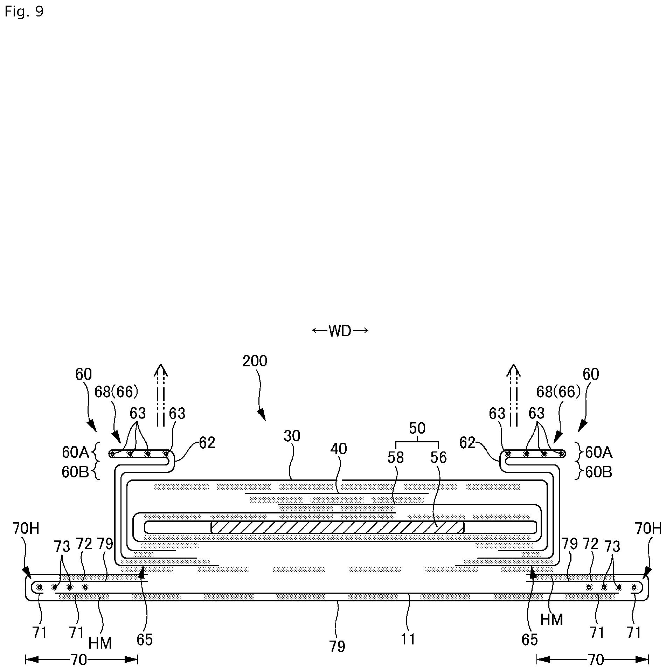

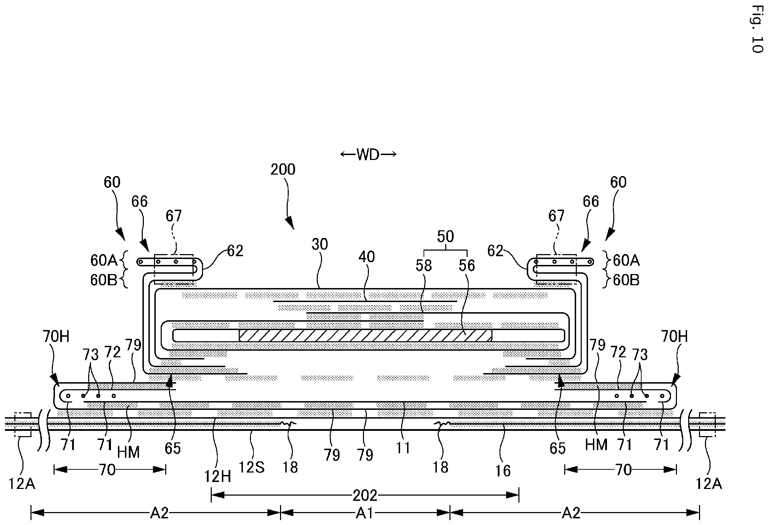

The sheet material forming the first sheet layer 71 and the second sheet layer 72 is not particularly limited, and it is possible to select an appropriate nonwoven fabric such as a nonwoven fabric that can be used in the rising gathers 60 and the outer bodies 12F and 12B described above. In the examples illustrated in Figs. 3, 4 and 11, the gather sheet 62 of the rising gather 60 is extended to form the first sheet layer 71 and the second sheet layer 72 as described later. As illustrated in Figs. 9, 10 and 12, a side flap 70 may be constructed by adding a dedicated sheet material 79 different from the rising gathers 60. In the former case, the front and back ends of the side flaps 70 coincide with the front and back ends of the rising gathers 60 (that is, the front and back ends of the inner body 200 in this case), but in the latter case, the front and back ends of the side flap 70 may be located on the center side in the front-back direction from the front and back ends of the rising gather 60.

The side elastic member 73 is not particularly limited, and the same elongated elastic member as the gather elastic member 63 can be used. The stretch rate of the side elastic member 73 in the side stretchable region SG in the unfolded state is preferably 150 to 350%, more preferably 200 to 270%. The number of side elastic members 73 is preferably 2 to 16, and more preferably 6 to 10. An appropriate arrangement interval of the side elastic members 73 is 5 to 10 mm. In particular, when a plurality of the side elastic members 73 is provided at intervals in the width direction WD, if the stretch rate of the side elastic members 73 in the side stretchable region SG in the unfolded state is lower toward the side, the side stretchable region SG rises slowly to the skin side, and the side stretchable region SG is likely to have an appropriately wearing state, which is preferable. If the side stretchable region SG rises tightly, the side flap 70 may be folded back inward and sandwiched between the leg and the surface of the disposable wearable article, and it may lead to leakage.

The side elastic member 73 is fixed to the first sheet layer 71 and the second sheet layer 72. For bonding the first sheet layer 71 and the second sheet layer 72, and for fixing the side elastic members 73 sandwiched therebetween, a hot melt adhesive HM by various application methods, a fixing means by material welding such as heat sealing and ultrasonic sealing can be used. If the bonding area of the first sheet layer 71 and the second sheet layer 72 is large, the flexibility is impaired. Therefore, it is preferable that the portion other than the adhesive portion of the side elastic member 73 is not bonded or is weakly bonded. In the illustrated example, by applying the hot melt adhesive HM only to the outer peripheral surface of the side elastic member 73 by application means such as a comb gun or a surewrap nozzle and sandwiching it between the first sheet layer 71 and the second sheet layer 72, only the hot melt adhesive HM applied to the outer peripheral surface of the side elastic member 73 is used to fix the side elastic member 73 to the first sheet layer 71 and the second sheet layer 72 and to fix the first sheet layer 71 and the second sheet layer 72.

The attachment range of the side elastic member 73 in the front-back direction LD, that is, the range of the side stretchable region SG in the front-back direction LD is from a portion overlapping the front outer body 12F to a portion overlapping the back outer body 12B. The range of the side stretchable region SG in the front-back direction LD is preferably the same as or wider than the range of the non-bonded portion 77 in the front-back direction LD. Further, it is also preferable that the range of the side stretchable region SG in the front-back direction LD be the same as the contracted portion of the rising gather 60 by the gather elastic member 63 or extending to the front and back sides from the contracted portion.

The side flap 70 preferably has three or more sheet layers including the first sheet layer 71 and the second sheet layer 72. That is, in addition to the most front surface side sheet layer 74 and the most back surface side sheet layer 75, it is preferable to have at least one inner sheet layer 76 located therebetween. A part or all of these sheet layers may be formed of separate sheet materials, or formed by folding a single sheet material once or a plurality of times. In addition to appropriately selecting the inner sheet layer 76 from the gather sheet 62 and the liquid impervious sheet 11 described above, or a nonwoven fabric similar to the outer bodies 12F and 12B described later, the inner sheet layer 76 can be formed by appropriately extending or folding the gather sheet 62 or the liquid impervious sheet 11 described above.

The structure of the hollow portion 70H is not particularly limited, but can be formed by, for example, the following laminated structure of sheet layers. That is, in the side flap 70 in the illustrated example, a non-bonded portion 77 is formed in which at least one of the most front surface side sheet layer 74 and the most back surface side sheet layer 75 and the inner sheet layer 76 overlapping with it are not bonded. This non-bonded portion 77 is a portion that continuously or intermittently continues in the front-back direction LD in the width direction WD range including the region between the side elastic member 73 located on the most side and the side edge of the side flap 70. That is, in the non-bonded portion 77, as long as either the most front surface side sheet layer 74 or the most back surface side sheet layer 75 and the inner sheet layer 76 that overlaps with it are not bonded, a part or all of the other layers may be bonded together, and all the sheet layers in the thickness direction may not be bonded. For example, in the non-bonded portion 77 of the examples illustrated in Figs. 3, 4 and 11, the portion where the most front surface side sheet layer 74 and the inner sheet layer 76 that overlaps with it is not bonded to each other continues from the side edge to the fixing position of the most lateral side elastic member 73, and the portion where the most back surface side sheet layer 75 and the inner sheet layer 76 that overlaps with it are not bonded to each other continues nearer to the center side in the width direction. In the non-bonded portion 77 of the examples illustrated in Figs. 9, 10, and 12, a portion where the most front surface side sheet layer 74 and the inner sheet layer 76 that overlaps with it are not bonded to each other and a portion where the most back surface side sheet layer 75 and the inner sheet layer 76 that overlaps with it are not bonded to each other continue from the side edge to the center side in the width direction with respect to the fixed position of the side elastic member 73 on the most side, but the former continues nearer to the center side in the width direction.

The dimension of the non-bonded portion 77 in the width direction WD can be appropriately determined, but is preferably 2 to 15 mm, and particularly preferably 5 to 10 mm. That is, the dimension of the hollow portion 70H in the width direction WD is preferably 2 mm or more. When the rising gather 60 is provided as in the illustrated example, it is preferable that the edge of the non-bonded portion 77 on the center side in the width direction WD be located more laterally than the boundary between the root portion 65 and the main unit section 66 (starting point of rising). Further, the dimension of the non-bonded portion 77 in the front-back direction LD (the dimension of the hollow portion 70H in the front-back direction) is preferably 30% or more, and particularly 40% or more of the maximum length Y of the product. Further, in the case of an underpants-type disposable wearable article in which the front outer body 12F and the back outer body 12B are separated from each other as in the illustrated example, it is preferable that the non-bonded portion 77 extend in the front-back direction LD to a position where the front outer body 12F and the back outer body 12B overlap each other. In this case, it is preferable that the non-bonded portion 77 extend over the entire front-back direction LD of the inner body 200. Further, it is also preferable that the non-bonded portion 77 extends only to a position between the front and back edges of the inner body 200 and the elastic members 16 and 19 closest to the leg opening LO side of the front outer body 12F and the back outer body 12B. Further, the non-bonded portion 77 may extend only to a position between the elastic members 16 and 19 closest to the leg opening LO in the front outer body 12F and the back outer body 12B and the edge of the leg opening LO (the back edge of the front outer body 12F and the front edge of the back outer body 12B).

On both sides of the non-bonded portion 77 in the width direction WD, the gap between at least one of the most front surface side sheet layer 74 and the most back surface side sheet layer 75 and the inner sheet layer 76 that overlaps with it is closed. As in the illustrated example, one side of the gap of the non-bonded portion 77 can be closed by folding back the sheet material forming the most front surface side sheet layer 74 or the most back surface side sheet layer 75 at the side edge of the side flap 70. Further, the other side of the gap of the non-bonded portion 77 can be closed by bonding the sheet layers adjacent to each other in the thickness direction at appropriate places by using a hot melt adhesive HM, a welding means or the like. It is obvious that, both sides of the gap of the non-bonded portion 77 can be closed by the same method, for example, the folding of the sheet material or the hot melt adhesive HM.

As illustrated in the example, a non-bonded portion 77 that is closed on both sides in the width direction WD is formed, and when this non-bonded portion 77 is continuous in the front-back direction LD in the width direction WD range (width direction WD range equal to or greater than the region) including the region between the side elastic member 73 located on the most side and the side edge of the intermediate region L, the hollow portion 70H surrounded by the sheet layer extends in the front-back direction LD on the side edge of the side flap. In this case, the pleats formed on the side portions of the side flaps are formed by at least one of the most front surface side sheet layer 74 and the most back surface side sheet layer 75 being greatly expanded, and are large and soft (that is, rich in cushioning properties). Therefore, since the large and soft pleats are formed inside, outside, or both inside and outside the range including the side edge of the side flap 70, or in the entire thickness direction, the touch feeling of the end portions of the leg opening LO is improved.

The side elastic member 73 may be located near the side edge of the side flap 70, but it is preferable that the side flap 70 do not include the side elastic member 73 in a portion of 2 to 15 mm (particularly 5 to 10 mm) from the side edge to the center side in the width direction WD. Further, it is preferable that a portion of 2 to 15 mm (particularly 5 to 10 mm) from the side edge to the center side in the width direction WD include a part or the whole of the hollow portion 70H. As described above, the side elastic member 73 is sufficiently separated from the side edge of the side flap 70, and a large pleat that is rich in flexibility and cushioning is formed in the entire thickness direction on the side of the portion that is pressed against the skin (that is, the part with the side elastic member 73) when the product is held by hand for wearing or purchasing, or during wearing, and it is particularly preferable in improving the touch feeling of the ends of leg openings LO. This effect is particularly remarkable when the side elastic member 73 is sufficiently separated from the side edge of the side flap 70, and the separated portion includes part or all of the hollow portion 70H.

The positional relationship between the hollow portion 70H and the side elastic member 73 can be appropriately determined, but at least one side elastic members 73 is provided in the portion located on the front surface side of the hollow portion 70H as in the illustrated example, the contraction force of the side elastic member 73 is directly applied to the hollow portion 70H, and it is preferable because the shape maintaining property of the pleats in the hollow portion 70H is improved.

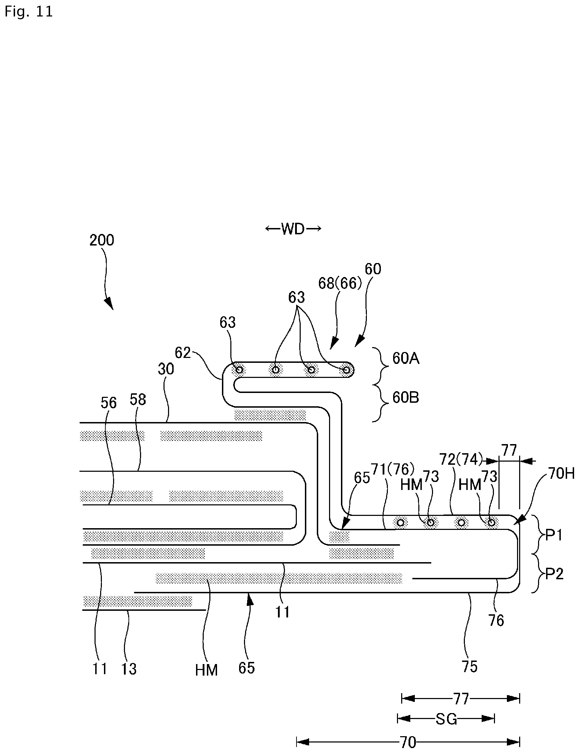

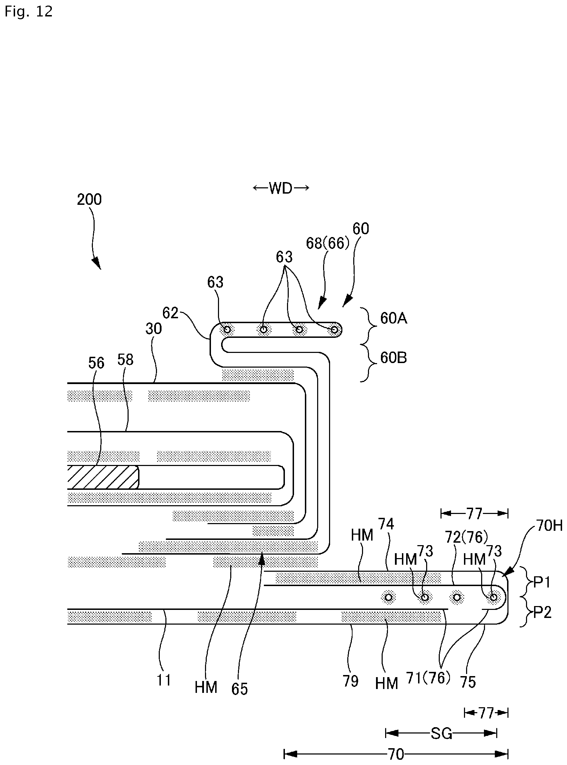

At the non-bonded portion 77, the inner sheet layer 76 overlapping with the most front surface side sheet layer 74 or the most back surface side sheet layer 75 may be fixed to the adjacent sheet layer on the opposite side as illustrated in Fig. 12 (the first sheet layer 71 and the second sheet layer 72 in the illustrated example are adhered to each other with a hot melt adhesive HM), and it is particularly preferable that the inner sheet layer 76 be not fixed to the sheet layers adjacent on both sides in the thickness direction as illustrated in Fig. 11. That is, it is preferable that a plurality of the hollow portions 70H defined by the inner sheet layer 76 be provided so as to overlap in the thickness direction. As a result, the pleats are independently formed in the portion located between the multiple hollow portions 70H, and the pleats are overlapped with each other such that the pleats having a better cushioning property are formed on the side portions of the side flaps 70.

As in the illustrated example, the side flap 70 has a folded-back sheet layer that is folded back at the side edge of the side flap 70 from the portion located on the front surface side of the hollow portion 70H to the portion located on the back surface side of the hollow portion 70H, and when the hollow portion 70H extends laterally to the folded back position of the folded-back sheet layer, it is preferable because the cushioning property of the pleats formed in the hollow portion 70H is particularly improved by the restoring force against the folding back. Note that the folded-back sheet layer means a pair of layers formed by folding the sheet material.

One preferred example is the structure illustrated in Figs. 3, 4, and 11. This side flap 70 has a double folded-back sheet layer folded back at its side edge, and the most front surface side sheet layer 74 and the most back surface side sheet layer 75 of the non-bonded portion 77 are a front surface side portion and a back surface side portion with respect to the folding position of the folded-back sheet layer located outside, respectively. Further, the side elastic member 73 is fixed at a position farther to the center side in the width direction WD than the folding position between the front surface side portion with the folding position of the folded-back sheet layer located outside as a boundary and the front surface side portion with the folding position of the folded-back sheet layer located inside as a boundary. Then, between the side elastic members 73 located on the most lateral side and the side edges of the side flaps 70, since all of the sheet layers (four layers) adjacent to each other in the thickness direction are not bonded, the hollow portions 70H are formed between the respective sheet layers. In this structure, it is possible to form hollow portions 70H in three tiers (three chambers in the thickness direction) in at least the side portions of the side flap 70. In this case, since each of the double folded-back sheet layers has a restoring force against folding back, a pleat having a superior cushioning property is formed on the side portion of the side flap 70 with less sheet material.

Another preferred example is the structure illustrated in Figs. 9, 10, and 12. This side flap 70 has a double folded-back sheet layer folded back at its side edge, the first sheet layer 71 and the second sheet layer 72 sandwiching the side elastic member 73 respectively include a portion on one side and a portion on the other side with the folding position of the folded-back sheet layer positioned inside as a boundary (that is, both the first sheet layer 71 and the second sheet layer 72 are the inner sheet layer 76). Then, the non-bonded portion 77 is a portion in which the most front surface side sheet layer 74 and the first sheet layer 71 overlapping with it are not bonded and a portion in which the most back surface side sheet layer 75 and the second sheet layer 72 overlapping with it are not bonded, and the hollow portion 70H laterally extends to the folded back position of the folded-back sheet layer located outside. In this structure, the hollow portions 70H are formed on both the front and back surface sides of the side portion of the side flap 70 portion, and as a result, large pleats are formed on both the front and back surface sides, such that the touch feeling of the side portions of the side flap 70 portion is improved. Further, since the side elastic member 73 is doubly covered with the folded-back sheet layer, the touch feeling of the side elastic member 73 is less likely to be transmitted to the skin, and the touch feeling is particularly improved. Furthermore, since the most front surface side sheet layer 74 and the most back surface side sheet layer 75 are a portion on one side and a portion on the other side with the folding position of the folded-back sheet layer located outside being sandwiched, due to the restoring force against the folding back, the pleats formed in the portion having the hollow portion 70H also have excellent cushioning properties.

Although not illustrated, the positions of the side elastic members 73 in the structures illustrated in Figs. 3, 4, and 11 may be located between the back surface side portion with the folding position of the folded-back sheet layer positioned outside as a boundary and the back surface side portion with the folding position of the folded-back sheet layer positioned inside as a boundary. That is, the side elastic member 73 may be attached to a portion located on the back surface side of the hollow portion 70H.

As in these examples, when the non-bonded portion 77 has a four-layered structure of sheet layers, as in the example illustrated in Fig. 11, it is preferable to construct the side flaps 70 by using a sheet of the rising gather 60. That is, the folded-back sheet layer of the side flap 70 described above can be formed by a first part P1 where the two-layered structure of the rising gather 60 sheet extends from the root portion 65 of the rising gather 60 to the side edge of the side flap 70 and a second part P2 in which the two-layered structure of the rising gather 60 sheet is folded back at the side edge of the side flap 70 and extends toward the center side in the width direction WD.

### (Outer Body)

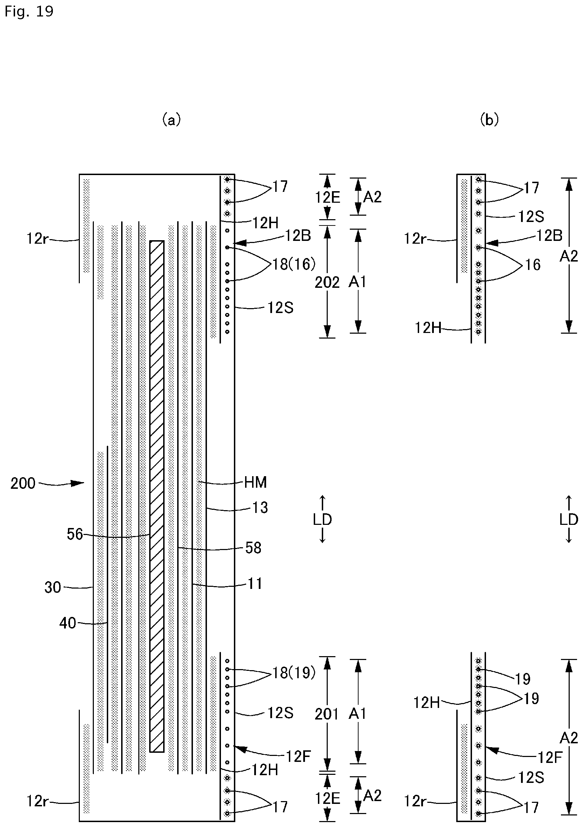

The outer bodies 12F and 12B are formed of a rectangular front outer body 12F that is a portion that forms at least the lower torso portion of the front body F, and a back outer body 12B that is a portion that forms at least the lower torso portion of the back body B. The front outer body 12F and the back outer body 12B are not continuous on the crotch side and are separated in the front-back direction LD (outer two-piece type). The distance in the front-back direction can be set to, for example, about 40 to 60% of the maximum length Y. In the illustrated example, the lower edges of the front outer body 12F and the back outer body 12B are linear along the width direction WD, the lower edges of at least one of the front outer body 12F and the back outer body 12B may be curved so as to extend around the legs. In addition, as illustrated in Fig. 19, the outer bodies 12F and 12B may be an integral body that extends continuously from the front body F to the back body B through the crotch (outer integral type).

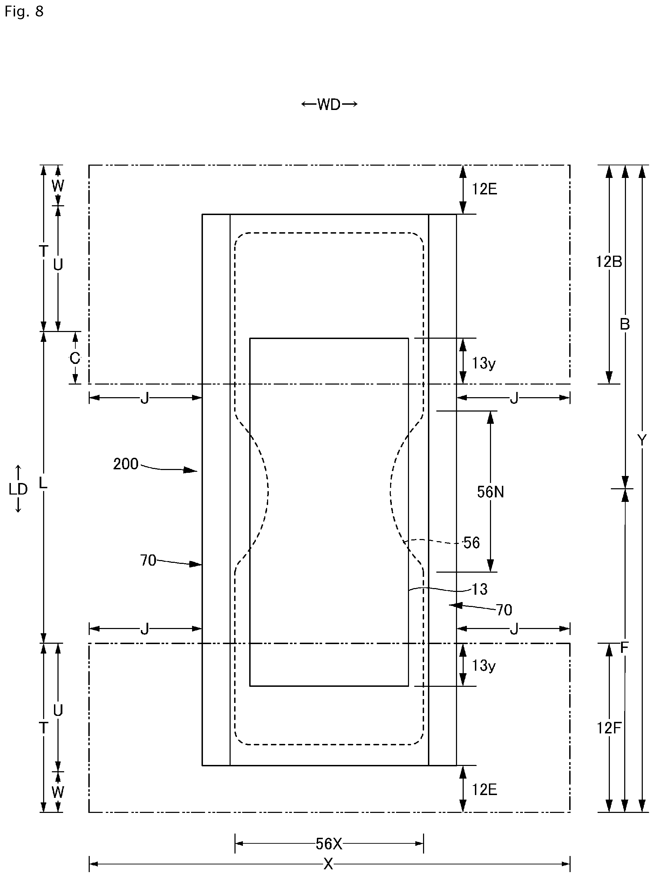

The outer bodies 12F and 12B have a lower torso portion which is a front-back direction range corresponding to the lower torso region T. Further, in this example, the front outer body 12F and the back outer body 12B have the same dimension in the front-back direction. As illustrated in Fig. 8, the structure is applied in which the back outer body 12B is longer than the front outer body 12F in the front-back direction, the back outer body 12B is provided with a gluteal cover portion extending from the lower torso region T to the intermediate region L side, and no portion corresponding to the intermediate region L is provided on the front outer body 12F. In this case, or conversely, although not illustrated, the front outer body 12F can also be provided with a groin cover portion extending from the lower torso region T to the intermediate region L side.

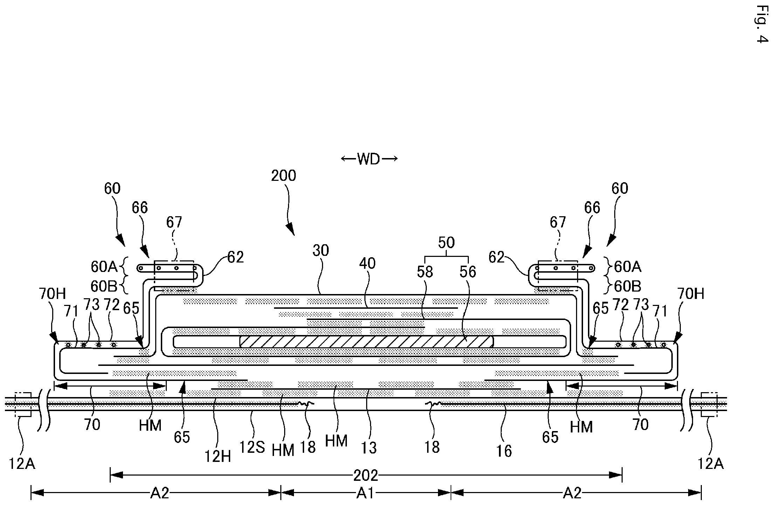

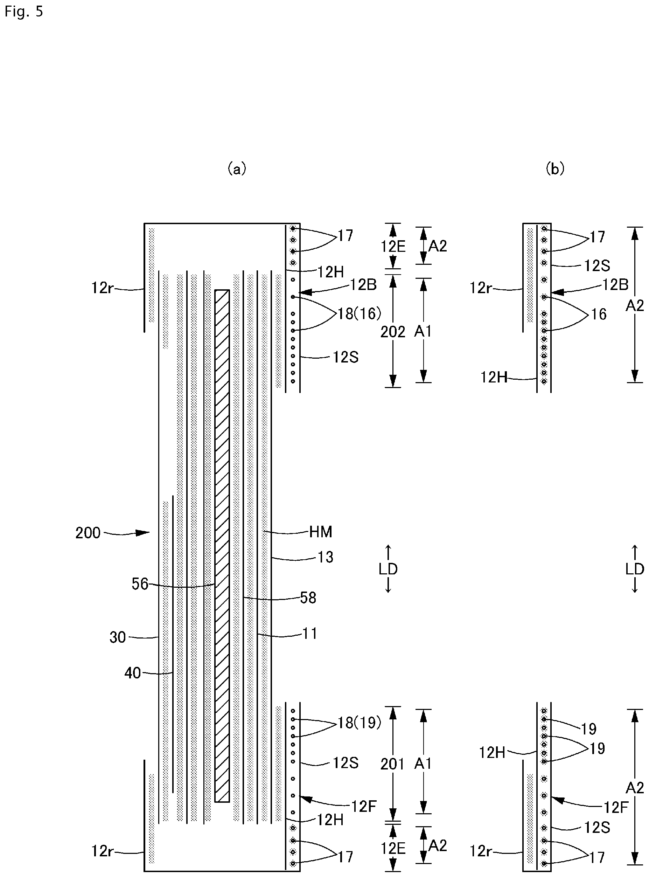

As illustrated in Figs. 4 and 5, the outer bodies 12F and 12B have an inner sheet layer 12H and an outer sheet layer 12S, and the elastic members 16 to 19 provided therebetween. Each sheet layer may be a common single sheet material or an individual sheet material. That is, in the former case, in part or all of the front outer body 12F and the back outer body 12B, the elastic members 16 to 19 can be provided between the inner portion and the outer portion of one sheet material folded back at appropriate positions such as the edge of the waist opening WO and the edge on the leg opening LO side. In the illustrated example, the waist portion W includes the folded-back portion of the sheet material. For example, in the waist portion W, although the sheet material forming the outer sheet layer 12S extends only up to the edge of the waist opening WO, but the sheet material forming the inner sheet layer 12H wraps around the waist side edge of the sheet material forming the second sheet layer and is folded back inside. Further, this folded-back portion is an inner cover layer 12r extending over the entire width direction of the outer body to a position where overlaps with the end of the inner body 200 on the waist opening WO side. The inner cover layer 12r may be formed by attaching a dedicated sheet material without folding back the sheet material forming the inner sheet layer 12H.

The elastic members 16 to 19 are incorporated in the outer bodies 12F and 12B to enhance fitting to the wearer's waist, and a stretchable region A2 which elastically expands and contracts in the width direction WD is formed accompanying expansion and contraction of the elastic member. In this stretchable region A2, the outer bodies 12F and 12B contract in accordance with the contraction of the elastic member in a natural length state, and wrinkles or pleats are formed, and when being stretched in the longitudinal direction of the elastic member, the outer bodies 12F and 12B can be stretched up to a predetermined stretch rate at which they can be stretched without wrinkles. As the elastic members 16 to 19, known elastic members such as a belt-shaped elastic member, a net-shaped elastic member, and a film-shaped elastic member can be used without particular limitation, in addition to the elongated elastic member (illustrated example) such as a rubber thread. As the elastic members 16 to 19, synthetic rubber, such as spandex, or natural rubber may be used.

When the elastic members 16 to 19 in the illustrated example is described in detail, in the waist portion W of the outer bodies 12F and 12B, a plurality of waist elastic members 17 is attached at intervals in the front-back direction so as to be continuously extend over the width direction WD. One or a plurality of waist elastic members 17 disposed in a region adjacent to the under-waist portion U may overlap with the inner body 200 or may be provided on both sides in the width direction except for the central portion in the width direction overlapping with the inner body 200. As this waist elastic members 17, rubber threads having a fineness of 155 to 1880 dtex, particularly about 470 to 1240 dtex (in the case of synthetic rubber) (in the case of natural rubber, a cross section area of about 0.05 to 1.5 mm², particularly about 0.1 to 1.0 mm²) are preferably provided at an interval of 2 to 12 mm, particularly at an interval of 3 to 7 mm, about 2 to 15 pieces, particularly about 4 to 10 pieces. As a result, the stretch rate in the width direction WD of the waist portion W is 150 to 400%, and particularly preferably about 220 to 320%. Further, it is not necessary to use the waist elastic member 17 having the same fineness or to set the same stretch rate in the entire front-back direction LD of the waist portion W. For example, at the upper portion and the lower portion of the waist portion W, the fineness and the stretch rate of the elastic member 17 may be different.

In addition, it is preferable that a plurality of under-waist elastic members 15 and 19 made of elongated elastic members is attached to the under-waist portion U of the outer bodies 12F and 12B at intervals in the front-back direction. As the under-waist elastic members 15 and 19, about 5 to 30 rubber threads having a fineness of 155 to 1880 dtex, particularly about 470 to 1240 dtex (in the case of synthetic rubber. In the case of natural rubber, a cross section area of about 0.05 to 1.5 mm², particularly about 0.1 to 1.0 mm²) are preferably provided at an interval of 1 to 15 mm, particularly 3 to 8 mm, such that a stretch rate of the width direction WD of the under-waist portion U is preferably about 200 to 350%, particularly about 240 to 300%.

Further, as illustrated in Fig. 8, when the back outer body 12B is provided with a gluteal cover portion as described above, and when a groin cover portion (not illustrated) is provided on the front outer body 12F, an elastic member similar to the under-waist portion can be provided, and as a result, the stretch rate in the width direction WD can be set to 150 to 300%, particularly 180 to 260%.

When the elastic members 16, and 19 are provided in the front-back direction range having the absorber 56 like the under-waist portion U in the illustrated example, to prevent contraction of the absorber 56 in the width direction WD in part or the whole of the range, the intermediate portion in the width direction (preferably including the entire inner and outer bonded portions 201 and 202) including a part or the whole of the portion overlapping with the absorber 56 in the width direction WD is a non-stretchable region A1, and both sides in the width direction is a stretchable region A2, which is preferable. It is preferable that the waist portion W is formed as the stretchable region A2 over the entire width direction WD. However, similarly to the under-waist portion U, the non-stretchable region A1 may be provided in the middle in the width direction. A portion of the stretchable region A2 located in the front-back direction LD range having the side seal portion 12A is an under-waist lower stretchable region A3.

The stretchable region A2 and the non-stretchable region A1 as described above are formed by supplying the elastic members 15 to 17 and 19 between the inner sheet layer 12H and the outer sheet layer 12S, fixing the elastic members 16, 19 with the hot melt adhesive only in the portion located in the stretchable region A2, in the region having the absorber 56, and cutting the elastic members 16 and 19 by pressing and heating at one place in the middle of the width direction, or finely cutting almost all of the elastic members 16 and 19 by pressing and heating, such that the elasticity is remained in the stretchable region A2 while suppressing the elasticity in the non-stretchable region A1.

The sheet material forming the inner sheet layer 12H and the outer sheet layer 12S can be used without particular limitation, but a nonwoven fabric is preferable. When a nonwoven fabric is used, its basis weight per sheet is preferably about 10 to 30 g/m².

The elastic members 16 to 19 are fixed to the outer bodies 12F and 12B by the hot melt adhesive HM formed by various application methods. The inner sheet layer 12H and the outer sheet layer 12S are preferably bonded by a hot melt adhesive HM for fixing the elastic members 16 to 19 at the portions having the elastic members 16 to 19, respectively, but in the portion not having the elastic members 16 to 19, they may be bonded by a hot melt adhesive HM or by material welding such as heat sealing or ultrasonic sealing, or part or all may not be adhered. At a portion having the elastic members 16 to 19 in the outer bodies 12F and 12B in the illustrated example, by applying the hot melt adhesive HM only to the outer peripheral surfaces of the elastic members 16 to 19 by an application means such as a comb gun or a surewrap nozzle and sandwiching them between sheet layers, fixing of the elastic members 16 to 19 to the both sheet layers and fixing between the both sheet layers are performed only by the hot melt adhesive HM applied to the outer peripheral surfaces of the elastic members 16 to 19. The elastic members 16 to 19 may be fixed to both sheet layers only at both ends in the stretchable direction in the stretchable region A2.

### (Lower End Gather)

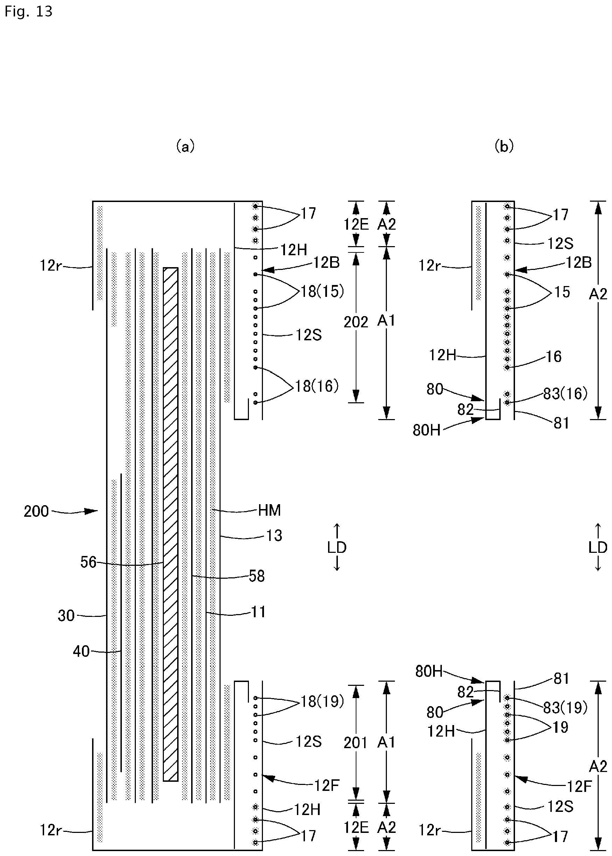

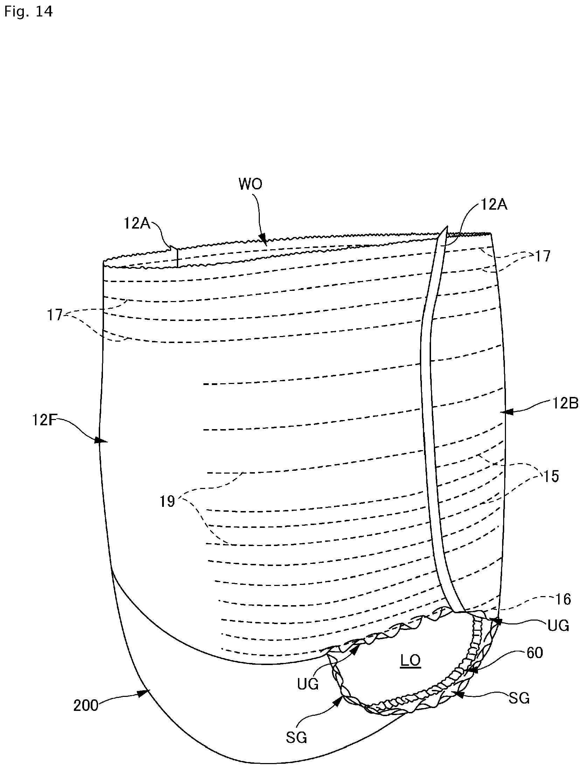

In the case of underpants-type disposable diapers with outer two-piece type, as illustrated in Figs. 13, 14, and 15, the side edge of the leg opening LO is the side flap 70 of the above-described inner body 200, and the upper edge of the leg opening LO is the lower edge of the wing region J that extends on both sides of the inner body 200 in the front outer body 12F and the back outer body 12B. The lower end portion 80 of the wing region J has a lower end stretchable region UG in which one (or a plurality of) elongated lower end elastic members 83 spaced from each other are provided along the width direction. Then, it is preferable that the lower end portion 80 of the wing region J has a hollow portion 80H continuing in the width direction along the lower edge thereof, and due to the contraction of the lower end elastic member 83, the part of the lower end portion 80 of the wing region J including the hollow portion 80H contracts in the width direction to form a lower end gather.

In the present disposable wearable article, the lower end elastic member 83 contracts, such that a portion including the hollow portion 80H along the lower edge of the wing region J contracts in the width direction WD to form the lower end gather. That is, the pleats of the lower end gathers are formed side by side in the width direction WD in a portion including the hollow portion 80H in the lower end portion of the wing region J. Here, when the lower end gather is formed in the portion including the hollow portion 80H in the wing region J, the pleats of the lower end gathers are formed by the hollow portion 80H being greatly expanded, and are large and soft (that is, rich in cushioning properties). Therefore, combined with the fact that the touch feeling at the lower end portion 80 of the wing region J of the outer bodies 12F and 12B improves (becomes soft and fluffy), and that the touch feeling at the above-described side portions of the side flaps 70 improves, the touch feeling of the entire edge of the leg opening LO improves.

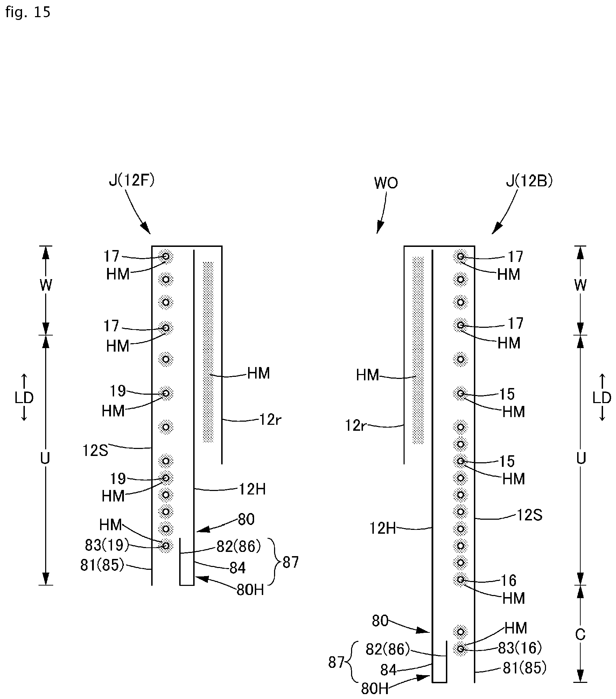

The structure of the hollow portion 80H is not particularly limited, but can be formed by, for example, the following laminated structure of sheet layers. That is, in the example illustrated in Fig. 15, the above-described second sheet material 12H is folded back to the outside at the edge of the leg opening LO, and the portion outside the folding position becomes the first sheet layer 81 facing the front surface side of the lower end elastic member 83, and a portion inside the folding position is the second sheet layer 82 facing the back surface side of the lower end elastic member 83. In place of this, appropriate changes are possible, such as that the first sheet material 12S is folded back at the edge of the leg opening LO to form the first sheet layer 81 and the second sheet layer 82, and the first sheet layer 81 is formed on one of the first sheet material 12S and the second sheet material 12H, and the second sheet layer 82 is formed on the other. Without using the above-described first sheet material 12S and second sheet material 12H as the first sheet layer 81 and the second sheet layer 82, it is also possible to add a sheet material forming the first sheet layer 81 and the second sheet layer 82 to the lower end portion 80 of the outer body 12F and 12B, and as the sheet material, an appropriate nonwoven fabric such as the nonwoven fabric that can be used for the rising gathers 60 and the outer bodies 12F and 12B described above can be selected.

In the case of the illustrated example, the lower end elastic member 83 is the under-waist elastic member 19 in the front outer body 12F and the cover elastic member 16 in the back outer body 12B, but is not limited to this, and it is also possible to provide a dedicated elastic member that is different from these in materials, stretch rate, arrangement, and the like. The stretch rate of the lower end elastic member 83 in the attached state is preferably 200 to 350%, more preferably 240 to 300%. An appropriate arrangement interval of the lower end elastic members 83 is 5 to 10 mm.

The lower end elastic member 83 in the illustrated example is fixed to the first sheet layer 81 and the second sheet layer 82. For bonding the first sheet layer 81 and the second sheet layer 82, and for fixing the lower end elastic members 83 sandwiched therebetween, a hot melt adhesive HM by various application methods, a fixing means by material welding such as heat sealing and ultrasonic sealing can be used. If the bonding area of the first sheet layer 81 and the second sheet layer 82 is large, the flexibility is impaired. Therefore, it is preferable that the portion other than the adhesive portion of the lower end elastic member 83 be not bonded or be weakly bonded. In the illustrated example, by applying the hot melt adhesive HM only to the outer peripheral surface of the lower end elastic member 83 by application means such as a comb gun or a surewrap nozzle and sandwiching it between the first sheet layer 81 and the second sheet layer 82, only the hot melt adhesive HM applied to the outer peripheral surface of the lower end elastic member 83 is used to fix the lower end elastic member 83 to the first sheet layer 81 and the second sheet layer 82 and to bond the first sheet layer 81 and the second sheet layer 82. Further, if the hot melt adhesive HM applied to the outer peripheral surface of the lower end elastic member 83 is simply used, and the bonding between the first sheet layer 81 and the second sheet layer 82 is insufficient, the hot melt adhesive HM can be appropriately added to a place where the lower end elastic member 83 is not provided.

The attachment range of the lower end elastic member 83 in the width direction, that is, the range of the lower end stretchable region UG in the width direction WD can be appropriately determined. When the non-stretchable region A1 is provided as in the illustrated example, the lower end elastic member 83 may be provided only on both sides in the width direction WD of the non-stretchable region A1, and although it is provided over the entire width direction WD, elasticity may be suppressed by cutting in the intermediate portion of the width direction WD.

The lower end portion 80 of the wing region J in the illustrated example has three or more sheet layers including the first sheet layer 81 and the second sheet layer 82. That is, in addition to the most front surface side sheet layer 84 and the most back surface side sheet layer 85, at least one inner sheet layer 86 located therebetween is provided. A part or all of these sheet layers may be formed of separate sheet materials, or formed by folding a single sheet material once or a plurality of times. The inner sheet layer 86 may be formed by directly or appropriately folding back the first sheet material 12S and the second sheet material 12H, or may be formed by adding a dedicated sheet material to the lower end portions 80 of the outer bodies 12F and 12B. As the dedicated sheet material in the latter case, an appropriate nonwoven fabric such as a nonwoven fabric that can be used in the rising gathers 60 and the outer bodies 12F and 12B described above can be selected.

The lower end portion 80 of the wing region J has a non-bonded portion 87 in which at least one of the most front surface side sheet layer 84 and the most back surface side sheet layer 85 and the inner sheet layer 86 which overlaps with the back side thereof are not bonded. That is, in the non-bonded portion 87, as long as either the most front surface side sheet layer 84 or the most back surface side sheet layer 85 and the inner sheet layer 86 that overlaps with it are not bonded, a part or all of the other layers may be bonded together, and all the sheet layers in the thickness direction may not be bonded.

In addition, the non-bonded portion 87 is a portion that continuously or intermittently continues in the width direction WD in the front-back direction LD range including the region between the lower end elastic member 83 located at the lowermost position and the lower edge of the wing region J. The dimension of the non-bonded portion 87 in the front-back direction LD can be appropriately determined, but is preferably 2 to 15 mm, and particularly preferably 5 to 10 mm. Further, the dimension of the non-bonded portion 87 in the width direction WD is preferably 50% or more, particularly preferably 90% or more of the dimension of the wing region J in the width direction WD.

In particular, in the case of an underpants-type disposable wearable article in which the front outer body 12F and the back outer body 12B have a non-stretchable region A1 and a stretchable region A2 area as in the illustrated example, the laminated structure of the wing region J (including the non-bonded portion) may be provided over the entire width of the product or may be provided only in the stretchable region A2.

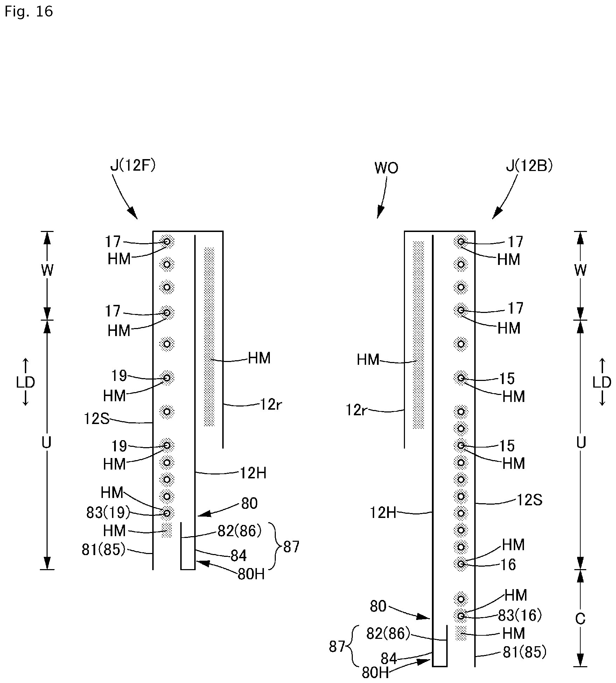

Furthermore, in the illustrated example, in the non-bonded portion 87, the hollow portion 80H is formed by closing a gap between at least one of the most front surface side sheet layer 84 and the most back surface side sheet layer 85 and the inner sheet layer 86 that overlaps with it on both sides of the non-bonded portion 87 in the front-back direction LD. As in the illustrated example, one side of the gap of the non-bonded portion 87 is closed by folding back the sheet material forming the most front surface side sheet layer 84 or the most back surface side sheet layer 85 at the lower edge of the wing region J, and the other side can be closed by appropriately adhering the sheet layer using a hot melt adhesive HM or a welding means. It is obvious that, both sides of the gap of the non-bonded portion 87 can be closed by the same method, for example, the folding of the sheet material or the hot melt adhesive HM. At the lower end portion 80 of the outer bodies 12F and 12B of the example illustrated in Figs. 15 and 16, the most back surface side sheet layer 85 has a portion that is not bonded to the inner sheet layer 86 that overlaps with it, but since the gap is not closed on the lower side, the gap is not the non-bonded portion 87 closed on both sides in the front-back direction LD, and a pleat having excellent cushioning properties described later is not formed.

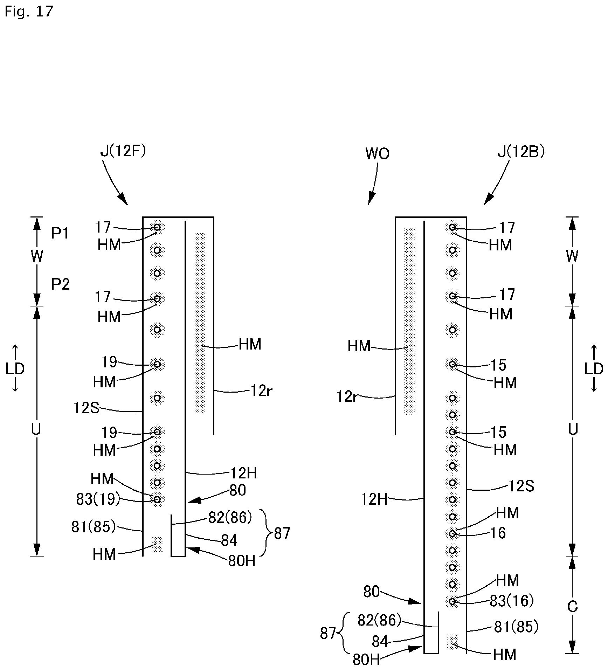

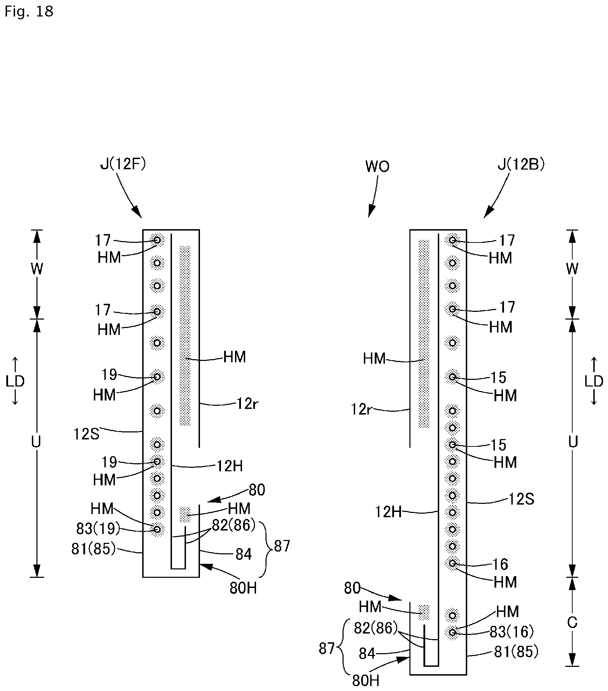

As can be seen from the above description, for example, in the non-bonded portion 87 of the example illustrated in Fig. 15, the portion where the most front surface side sheet layer 84 and the inner sheet layer 86 that overlaps with it is not bonded to each other continues from the lower edge of the outer bodies 12F and 12B to the fixing position of the lowermost lower end elastic member 83. Further, in the non-bonded portion 87 of the example illustrated in Fig. 16 and the example illustrated in Fig. 17, the portion where the most front surface side sheet layer 84 and the inner sheet layer 86 that overlaps with it is not bonded to each other continues from the lower edge of the outer bodies 12F and 12B to the upper edge of the inner sheet layer 86. Further, in the non-bonded portion 87 in the example illustrated in Fig. 18, the portion where the most back surface side sheet layer 85 and the inner sheet layer 86 that overlap with it are not bonded to each other continues from the lower edge of the outer bodies 12F and 12B to the fixing position of the lowermost lower end elastic member 83, and the portion where the most front surface side sheet layer 84 and the inner sheet layer 86 that overlaps with it are not bonded to each other continues from the lower edge of the outer bodies 12F and 12B to the upper edge of the inner sheet layer 86.

The non-bonded portion 87 is a portion where at least one of the most front surface side sheet layer 84 and the most back surface side sheet layer 85 and the inner sheet layer 86 overlapping with it are not bonded, and a gap between the sheet layers is closed on both sides of the non-bonded portion 87 in the front-back direction LD. Further, the non-bonded portion 87 continues in the width direction WD in the front-back direction LD range including the region between the lower end elastic member 83 located at the lowest position and the lower edge of the wing region J (range of the front-back direction LD equal to or greater than the region). That is, the hollow portion surrounded by the sheet layer continues to the non-bonded portion 87 in the width direction. As a result, the pleats formed on the non-bonded portion 87 are formed by at least one of the most front surface side sheet layer 84 and the most back surface side sheet layer 85 being greatly expanded, and are large and soft (that is, rich in cushioning properties). Therefore, since the large and soft pleats are formed inside, outside, both inside and outside the range including the lower edge of the wing region J or in the entire thickness direction, the touch feeling of the end portions of the leg openings LO in the outer bodies 12F and 12B is improved.

The lower end elastic member 83 may be located near the lower edge of the wing region J, but the portion from the lower edge of the wing region J to the center side in the front-back direction LD of 2 to 15 mm (particularly 5 to 10 mm) preferably does not include the lower end elastic member 83 and include a part or all of the hollow portion 80H. As described above, the lower end elastic member 83 is sufficiently separated from the lower edge of the wing region J, and if the separated portion includes a part or all of the hollow portion 80H, when the product is held by hand for wearing or purchasing, or during wearing, a large pleat that is rich in flexibility and cushion is formed in the entire thickness direction on the lower side of the portion that is pressed against the skin (that is, the part with the lower end elastic member 83). Therefore it is particularly preferable in improving the touch feeling of the ends of leg openings LO.

The positional relationship between the hollow portion 80H and the lower end elastic member 83 can be appropriately determined, but as illustrated in the example, when at least one lower end elastic member 83 is provided at a position overlapping the hollow portion 80H in the thickness direction, it is preferable because the contraction force of the lower end elastic member 83 is directly applied to the non-bonded portion 87, and the shape-retaining property of the pleats in the non-bonded portion 87 is improved.

At the non-bonded portion 87, as illustrated in Figs. 15 and 16, the inner sheet layer 86 may be fixed to the sheet layer adjacent in one of the thickness direction, but as illustrated in Figs. 17 and 18, it is also preferable that the inner sheet layer 86 is not fixed to the adjacent sheet layers on both sides in the thickness direction. Thereby, the pleats are independently formed on the inner sheet layer 86 by the contraction force of the lower end elastic member 83. That is, since a largely expanding pleat formed on the lower end portion 80 of the wing region J is supported by the pleats of the inner sheet layer 86 independently formed on the inside, a pleat having more excellent cushioning property is formed at the lower end portion 80 of the wing region J.

As in the example illustrated in Figs. 15 to 17, the second sheet material 12H has a portion forming the most front surface side sheet layer 84 and a portion forming the inner sheet layer 86, which is folded back to the side having the lower end elastic member 83 at the lower edge of the wing region J, and at least a part of the portion forming the inner sheet layer 86 is preferably bonded to the first sheet material 12S. On the contrary, the first sheet material 12S has a portion forming the most back surface side sheet layer 85 and a portion forming the inner sheet layer 86, which is folded back to the side having the lower end elastic member 83 at the lower edge of the wing region J, and at least a part of the portion forming the inner sheet layer 86 may be bonded to the second sheet material 12H (not illustrated). The inner sheet layer formed by folding back one of the first sheet material 12S and the second sheet material 12H and the other sheet material may be bonded, as in the example illustrated in Fig. 15, by using a hot melt adhesive HM for fixing the lower end elastic member 83 or by applying a hot melt adhesive HM separately as illustrated in Figs. 16 and 17. Further, the bonding position may be spaced apart from the lower edge of the wing region J to some extent (for example, about 5 to 10 mm) as in the example illustrated in Figs. 15 and 16, and the bonding position may be near the lower edge of the wing region J as in the example illustrated in Fig. 17. In this structure, the inner sheet layer 86 can be formed by a sheet material that forms one of the first sheet layer 81 and the second sheet layer 82 that sandwich the lower end elastic member 83, and also the portion forming the most front surface side sheet layer 84 or the most back surface side sheet layer 85 has a restoring force against the folding back of the portion forming the inner sheet layer 86, and therefore pleats having a superior cushioning property is formed on the lower end portion 80 of the wing region J with less sheet material.

The example illustrated in Fig. 18 is also preferable. In this structure, the first sheet material 12S and the second sheet material 12H have a folded-back portion that is folded back inward at the lower edge of the wing region J. Then, the most back surface side sheet layer 85 is a portion on one side and a portion on the other side across the folding position of the first sheet material 12S, and the most front surface side sheet layer 84 is a portion on one side and a portion on the other side across the folding position of the second sheet material 12H. The inner sheet layer 86 is a portion on one side and a portion on the other side across the folding position of the second sheet material 12H. In this structure, by folding back the first sheet material 12S and the second sheet material 12H sandwiching the lower end elastic member 83, two sheet layers other than the first sheet layer 81 and the second sheet layer 82 can be formed, and each has a restoring force against folding back. Therefore pleats having a superior cushioning property is formed in the lower end portion 80 of the wing region J with less sheet material. The folding directions of the first sheet material 12S and the second sheet material 12H may be outward, and in that case, basically the same advantages are provided except that the directions are different.

### (Inner and Outer Bonded Portion)

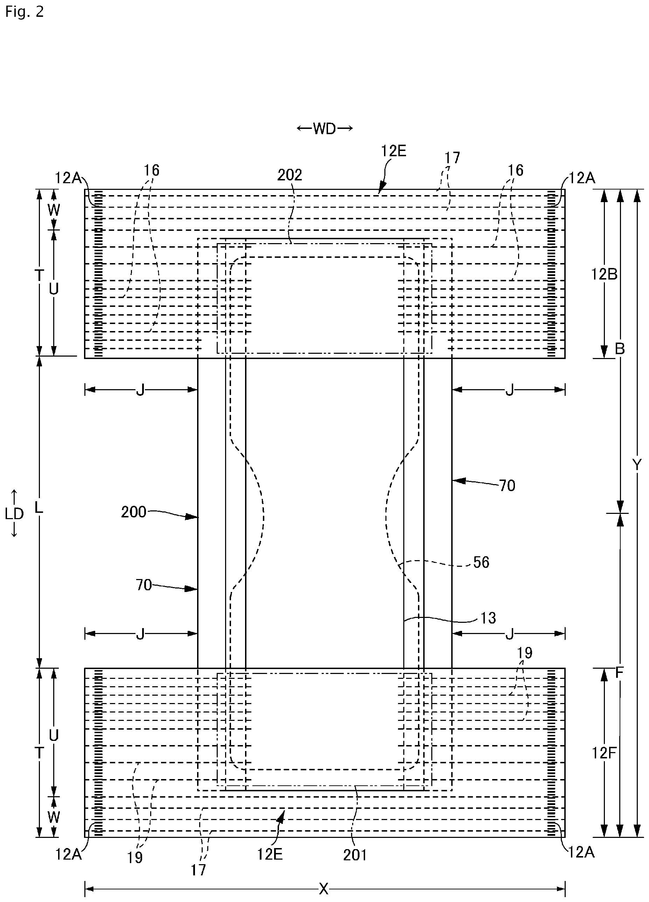

The inner body 200 can be fixed to the outer bodies 12F and 12B by a bonding means by material welding such as heat sealing or ultrasonic sealing or a hot melt adhesive. In the illustrated example, the inner body 200 is fixed to the inner surface of the outer bodies 12F and 12B via a hot melt adhesive applied to the back surface of the inner body 200, that is, in this case, the back surface of the liquid impervious sheet 11 and the root portion 65 of the rising gather 60. Inner and outer bonded portions 201 and 202 for fixing the inner body 200 and the outer bodies 12F and 12B can be provided almost entirely in a region where those are overlapped with each other, as illustrated in Fig. 2, and, for example, they can be provided in portions excluding both ends in the width direction of the inner body 200.

### (Cover Nonwoven Fabric)

In outer two-piece type underpants-type disposable diapers, since the inner body 200 is exposed between the front outer body 12F and the back outer body 12B, in order to prevent the liquid impervious sheet 11 from being exposed on the back surface of the inner body 200, it is preferable that, on the back surface of the inner body 200, the cover nonwoven fabric 13 from a space between the front outer body 12F and the inner body 200 to a space between the back outer body 12B and the inner body 200 be provided. The inner surface and the outer surface of the cover nonwoven fabric 13 can be adhered to the opposite surfaces via a hot melt adhesive. As the nonwoven fabric used for the cover nonwoven fabric 13, for example, the same material as that of the outer bodies 12F and 12B can be appropriately selected.

### (Nonwoven Fabric)

As the nonwoven fabric in the above description, a known nonwoven fabric can be appropriately used depending on the site or purpose. Examples of the constituent fibers of the nonwoven fabric include synthetic fibers (including not only single component fibers but also composite fibers such as a core-sheath) such as polyolefin-based such as polyethylene or polypropylene, polyester-based, and polyamide-based, regenerated fibers such as rayon and cupra, and natural fibers such as cotton, but it can be selected without limitation, and these can be mixed and used. In order to increase the flexibility of the nonwoven fabric, it is preferable that the constituent fibers be crimped fibers. Further, the constituent fibers of the nonwoven fabric may be hydrophilic fibers (including hydrophobic fibers rendered hydrophilic by a hydrophilizing agent), and may be hydrophobic fibers or water-repellent fibers (including water-repellent fibers rendered water-repellent by a water repellent agent). In addition, generally according to a fiber length, a sheet forming method, a fiber bonding method, and a laminated structure, the nonwoven fabric is classified into short fiber nonwoven fabric, long fiber nonwoven fabric, spun bond nonwoven fabric, melt blown nonwoven fabric, spun lace nonwoven fabric, thermal bond (air through) nonwoven fabric, needle punch nonwoven fabric, point bond nonwoven fabric, laminated nonwoven fabric (SMS nonwoven fabric, SMMS nonwoven fabric, etc. with a melt blown layer(s) sandwiched between spun bond layers), etc., but any of these nonwoven fabrics can be used.

### <Explanation of Terms Used Herein>

The following terms in the specification have the following meanings unless otherwise specified in the specification.

"Front-back direction" means the direction indicated by the reference character LD in the drawings (longitudinal direction), and "width direction" means the direction indicated by WD in the drawings (left-right direction), and the front-back direction and the width direction are orthogonal to each other.

"Front surface side" means a side close to the skin of a wearer wearing an underpants-type disposable diaper. "Back surface side" means a side far from the skin of a wearer wearing an underpants-type disposable diaper.

"Front surface" means a surface of a member close to the skin of a wearer wearing an underpants-type disposable diaper. "Back surface" means a surface far from the skin of a wearer wearing an underpants-type disposable diaper.

"Stretch rate" means the value when the natural length is taken as 100%. For example, a stretch rate of 200% is synonymous with a stretch magnification of 2 times.

"Gel strength" is measured as follows: A super absorbent polymer 1.0 g is added to artificial urine (mixture of urea: 2wt%, sodium chloride: 0.8wt%, calcium chloride dihydrate: 0.03wt%, magnesium sulfate heptahydrate: 0.08wt%, and ion exchanged water: 97.09wt%) 49.0 g, and stirred with a stirrer. After leaving generated gel for three hours in a thermohygrostat bath at 40°C × 60%RH, return to room temperature, and measure the gel strength with a curdmeter (Curdmeter-MAX ME-500, manufactured by I. techno Engineering).

"Basis weight" is measured as follows. After preliminary drying a sample or a test piece, the sample or the test piece is left in a test chamber or equipment in the standard state (the test location is at a temperature of 23 ± 1°C and with a relative humidity of 50 ± 2%) to be constant weight. The preliminary drying refers to making a sample or a test piece a constant weight in an environment at a temperature of 100°C. For fibers with an official moisture regain of 0.0%, preliminary drying may not be performed. A sample of dimensions of 100 mm × 100 mm is cut using a template for sampling (100 mm × 100 mm) from a test piece in a constant weight. The basis weight is set by weighing the sample, multiplying by 100, and calculating the weight per one square meter.

"Thickness" is automatically measured under the conditions of a load of 0.098 N/cm² and a pressing area of 2 cm² using an automatic thickness measuring device (KES-G5 handy compression measuring program).

"Water absorption capacity" is measured according to JIS K7223-1996 "Testing method for water absorption capacity of super absorbent polymers".

"Water absorptionspeed" is the "time to end point" when JIS K7224-1996 "Testing method for water absorption speed of super absorbent polymers" has been carried out using 2 g of super absorbent polymer and 50 g of physiological saline solution.

"Unfolded state" means a flatly spread state without shrinkage or slackness.

The dimension of each portion means the dimension in the unfolded state, not the natural length state, unless otherwise stated.

When environmental conditions in tests and measurements are not described, the tests and measurements shall be carried out in a test room or apparatus in a standard state (a temperature of 23 ± 1°C and a relative humidity of 50 ± 2% at the test location).

### Industrial Applicability

The present invention can be applied to disposable wearable articles in general, such as tape-type disposable diapers, pad-type disposable diapers, sanitary napkins, and the like, in addition to underpants-type disposable diapers.

### Reference Signs List

11 liquid impervious sheet
12A side seal portion
12B back outer body
12E waist extended section
12F front outer body
12F, 12B outer body
12H inner sheet layer
12S outer sheet layer
13 cover nonwoven fabric
15, 19 under-waist elastic member
17 waist elastic member
200 inner body
201, 202 inner and outer bonded portion
30 top sheet
40 intermediate sheet
50 absorbent element
56 absorber
58 package sheet
60 rising gather
60A tip-side portion
60B root-side portion
62 gather sheet
63 gather elastic member
67 fallen portion
68 rising portion
70 side flap portion
71 first sheet layer
72 second sheet layer
73 side elastic member
74 most front surface side sheet layer
75 most back surface side sheet layer
76 inner sheet layer
77 non-bonded portion
79 dedicated sheet material
80 lower end portion
83 lower end elastic member
70H, 80H hollow portion
A1 non-stretchable region
A2 stretchable region
A3 under-waist stretchable region
F front body
B back body
J wing region
L intermediate region
LD front-back direction
SG side stretchable region
UG lower end stretchable region
T lower torso region
U under-waist portion
W waist portion
WD width direction
WO waist opening
LO leg opening
P1 first part
P2 second part
HM hot melt adhesive

## Claims

1. A disposable wearable article, comprising:
a crotch portion; and
side flaps (70) without an absorber (56) provided on both side portions of the crotch portion,
the side flaps (70) having a side stretchable region (SG) in which one or a plurality of elongated side elastic members (73) spaced from each other is provided along a front-back direction (LD) of the disposable wearable article,
wherein each side flap (70) has a hollow portion (70H) continuing in the front-back direction (LD) along an outside edge of the side flap (70),
each side flap (70) has a double folded-back sheet layer folded back at the outside edge of the side flap (70), of which a most front surface side sheet layer (74) and a most back surface side sheet layer (75) in the side flap (70) are a front surface side portion and a back surface side portion, respectively, with the folding position of the folded-back sheet layer forming a boundary located at the outside edge,
each side elastic member (73) is fixed between a front surface side part of the outer layer of the double folded-back sheet layer and a front surface side part of the inner layer of the double folded-back sheet layer, and located at a position farther toward the center of the disposable wearable article in a width direction (WD) than the folding position,
all of the sheet layers adjacent to each other in the thickness direction are not bonded between the outermost side elastic member (73) and the outside edge of the side flap (70), thereby forming a hollow portion (70H) between each of the sheet layers, and
a part of the side flap (70) including the hollow portion (70H) contracts in the front-back direction (LD) due to contraction of the one or more side elastic members (73) to form a side gather.

2. The disposable wearable article according to claim 1,
wherein the hollow portion (70H) extends from the outside edge of the side flap (70) to a position separated from the outside edge by 2 mm or more toward the center of the disposable wearable article in the width direction (WD).

3. The disposable wearable article according to claims 1 or 2,
wherein a portion in the side flap (70) of 2 to 15 mm from the outside edge toward the center of the disposable wearable article in the width direction (WD) does not include a side elastic member (73), and includes a part or all of the hollow portion (70H).

4. The disposable wearable article according to any one of claims 1 to 3,
wherein at least one side elastic member (73) is provided on the front surface side of the hollow portion (70H).

5. The disposable wearable article according to any one of claims 1 to 4,
wherein rising gathers (60) rise up from a position on a center side in the width direction (WD) relative to the one or more side elastic members (73) and extend along the front-back direction (LD),
each rising gather (60) includes a gather sheet (62) that is folded in two at a tip portion to form a two-layered structure, and an elongated gather elastic member (63) is provided along at least the interlayer of the two-layered structure in the front-back direction (LD),
both side portions of each side flap (70) have a first part (P1) in which the two-layered structure made of the gather sheet (62) extends from a starting point to the outside edge of the side flap (70) and a second part (P2) in which the two-layered structure made of the gather sheet (62) is folded back at the outside edge of the side flap (70) and extends toward the center of the disposable wearable article in the width direction (WD), and
the double folded-back sheet layer is the first part (P1) and the second part (P2) of the gather sheet (62).

6. The disposable wearable article according to any one of claims 1 to 5,
wherein multiple side elastic members (73) are provided at intervals in the width direction (WD), and
a stretch rate of the side elastic member (73) in the side stretchable region (SG) in an unfolded state is lower as it is located laterally.

7. The disposable wearable article according to any one of claims 1 to 6,
which is an underpants-type disposable wearable article comprising an outer body (12F,12B) forming at least a lower torso portion of a front body (F) and a back body (B), an inner body (200) incorporating an absorber (56) and attached to the outer body (12F,12B) so as to extend from the front body (F) to the back body (B), a waist opening (WO), and a pair of left and right leg openings (LO),
wherein the side flaps (70) are provided on both side portions of the inner body (200).

## Patentansprüche

1. Wegwerfbarer, tragbarer Artikel, umfassend:
einen Schrittabschnitt; und
Seitenklappen (70) ohne einen Absorber (56), die an beiden Seitenabschnitten des Schrittabschnitts vorgesehen sind,
wobei die Seitenklappen (70) einen seitlichen dehnbaren Bereich (SG) aufweisen, in dem ein oder eine Vielzahl länglicher seitlicher elastischer Elemente (73) vorgesehen ist, die voneinander beabstandet sind, entlang einer Vorderseiten-Rückseiten-Richtung (LD) des wegwerfbaren, tragbaren Artikels,
wobei jede Seitenklappe (70) einen hohlen Abschnitt (70H) aufweist, der in der Vorderseiten-Rückseiten-Richtung (LD) entlang einer Außenkante der Seitenklappe (70) verläuft,
jede Seitenklappe (70) eine doppelte zurückgefaltete Schichtlage aufweist, die an der Außenkante der Seitenklappe (70) zurückgefaltet ist, von der eine vorderste Oberflächenseitenschichtlage (74) und eine hinterste Oberflächenseitenschichtlage (75) in der Seitenklappe (70) ein vorderer Oberflächenseitenabschnitt bzw. ein hinterer Oberflächenseitenabschnitt sind, wobei die Faltposition der zurückgefalteten Schichtlage eine an der Außenkante befindliche Grenze bildet,
jedes seitliche elastische Element (73) zwischen einem vorderen Oberflächenseitenteil der äußeren Schicht der doppelten zurückgefalteten Schichtlage und einem vorderen Oberflächenseitenteil der inneren Schicht der doppelten zurückgefalteten Schichtlage befestigt ist und sich an einer Position befindet, die in einer Breitenrichtung (WD) weiter zur Mitte des wegwerfbaren, tragbaren Artikels hin liegt als die Faltposition,
alle Schichtlagen, die in der Dickenrichtung zueinander benachbart sind, nicht zwischen dem äußersten seitlichen elastischen Element (73) und der Außenkante der Seitenklappe (70) verbunden sind, wodurch ein hohler Abschnitt (70H) zwischen jeder der Schichtlagen gebildet wird, und
ein Teil der Seitenklappe (70), der den hohlen Abschnitt (70H) umfasst, sich in der Vorderseiten-Rückseiten-Richtung (LD) aufgrund einer Kontraktion des einen oder der mehreren seitlichen elastischen Element (73) zusammenzieht, um eine Seitenfalte oder Seitenraffung zu bilden.

2. Wegwerfbarer, tragbarer Artikel nach Anspruch 1,
wobei sich der hohle Abschnitt (70H) von der Außenkante der Seitenklappe (70) bis zu einer Position erstreckt, die von der Außenkante um 2 mm oder mehr in Richtung der Mitte des wegwerfbaren, tragbaren Artikels in der Breitenrichtung (WD) separiert ist.

3. Wegwerfbarer, tragbarer Artikel nach Anspruch 1 oder 2,
wobei ein Abschnitt in der Seitenklappe (70) von 2 bis 15 mm von der Außenkante in Richtung auf die Mitte des wegwerfbaren, tragbaren Artikels in der Breitenrichtung (WD) kein seitliches elastisches Element (73) umfasst und einen Teil oder alles von dem gesamten hohlen Abschnitt (70H) umfasst.

4. Wegwerfbarer, tragbarer Artikel nach einem der Ansprüche 1 bis 3,
wobei mindestens ein seitliches elastisches Element (73) an der vorderen Oberflächenseite des hohlen Abschnitts (70H) vorgesehen ist.

5. Wegwerfbarer, tragbarer Artikel nach einem der Ansprüche 1 bis 4,
wobei aufsteigende Raffungen (60) von einer Position auf einer Mittenseite in der Breitenrichtung (WD) relativ zu dem einen oder den mehreren seitlichen elastischen Elementen (73) aufsteigen und sich entlang der Vorderseiten-Rückseiten-Richtung (LD) erstrecken,
jede aufsteigende Raffung (60) eine Raffungsschicht (62) umfasst, die an einem Spitzenabschnitt in zwei gefaltet ist, um eine zweischichtige Struktur zu bilden, und ein längliches elastisches Raffungselement (63) entlang mindestens der Zwischenschicht der zweischichtigen Struktur in der Vorderseiten-Rückseiten-Richtung (LD) vorgesehen ist,
beide Seitenabschnitte jeder Seitenklappe (70) einen ersten Teil (P1) aufweisen, in dem sich die zweischichtige Struktur, die aus der Raffungsschicht (62) gemacht ist, von einem Startpunkt bis zum Außenrand der Seitenklappe (70) erstreckt, und einen zweiten Teil (P2), in dem die zweischichtige Struktur, die aus der gerafften Lage (62) gemacht ist, am der Außenkante der Seitenklappe (70) zurückgefaltet ist und sich in Breitenrichtung (WD) zur Mitte des wegwerfbaren, tragbaren Artikels erstreckt, und
die doppelte zurückgefaltete Schichtlage der erste Teil (P1) und der zweite Teil (P2) der Raffungsschicht (62) ist.

6. Wegwerfbarer, tragbarer Artikel nach einem der Ansprüche 1 bis 5,
wobei mehrere seitliche elastische Elemente (73) in Intervallen in der Breitenrichtung (WD) vorgesehen sind, und
eine Dehnungsrate des seitlichen elastischen Elements (73) in dem seitlichen dehnbaren Bereich (SG) in einem ungefalteten Zustand geringer ist, als wenn es seitlich angeordnet ist.

7. Wegwerfbarer, tragbarer Artikel nach einem der Ansprüche 1 bis 6,
der ein wegwerfbarer, tragbarer Artikel vom Unterhosen-Typ ist, umfassend einen äußeren Körper (12F, 12B), der mindestens einen unteren Rumpfabschnitt eines vorderen Körpers (F) und eines hinteren Körpers (B) bildet, einen inneren Körper (200), der einen Absorber (56) umfasst und an dem äußeren Körper (12F, 12B) so angebracht ist, dass er sich von dem vorderen Körper (F) zu dem hinteren Körper (B) erstreckt, eine Taillenöffnung (WO) und ein Paar linke und rechte Beinöffnungen (LO),
wobei die Seitenklappen (70) an beiden Seitenabschnitten des inneren Körpers (200) vorgesehen sind.

## Revendications

1. Article portable jetable, comprenant : une partie d'entrejambe ; et
des rabats latéraux (70) sans absorbeur (56) disposés sur les deux parties latérales de la partie d'entrejambe,
les rabats latéraux (70) ayant une région latérale extensible (SG) dans laquelle un ou plusieurs éléments élastiques latéraux allongés (73) espacés l'un de l'autre sont disposés le long d'une direction avant-arrière (LD) de l'article portable jetable,
chaque rabat latéral (70) comportant une partie creuse (70H) se prolongeant dans la direction avant-arrière (LD) le long d'un bord extérieur du rabat latéral (70),
chaque rabat latéral (70) comporte une double couche de feuille repliée au niveau du bord extérieur du rabat latéral (70), dont une couche de feuille latérale de surface la plus à l'avant (74) et une couche de feuille latérale de surface la plus à l'arrière (75) dans le rabat latéral (70) sont respectivement une partie latérale de surface avant et une partie latérale de surface arrière, la position de pliage de la couche de feuille repliée formant une limite située au niveau du bord extérieur,
chaque élément élastique latéral (73) est fixé entre une partie latérale de surface avant de la couche extérieur de la couche de feuille arrière doublement pliée et une partie latérale de surface avant de la couche intérieure de la couche de feuille doublement pliée, et est situé à une position plus vers le centre de l'article portable jetable dans le sens de la largeur (WD) que dans la position de pliage,
toutes les couches de feuilles adjacentes les unes aux autres dans le sens de l'épaisseur ne sont pas liées entre l'élément élastique latéral le plus extérieur (73) et le bord extérieur du rabat latéral (70), formant ainsi une partie creuse (70H) entre chacune des couches de feuille, et
une partie du rabat latéral (70) comprenant la partie creuse (70H) se contracte dans la direction avant-arrière (LD) en raison de la contraction du ou des éléments élastiques latéraux (73) pour former une fronce latérale.

2. Article portable jetable selon la revendication 1, dans lequel la partie creuse (70H) s'étend depuis le bord extérieur du rabat latéral (70) vers une position séparée du bord extérieur de 2 mm ou plus vers le centre de l'article portable jetable dans le sens de la largeur (WD).

3. Article portable jetable selon les revendications 1 ou 2,
dans lequel une partie dans le rabat latéral (70) de 2 à 15 mm depuis le bord extérieur vers le centre de l'article portable jetable dans le sens de la largeur (WD) ne comprend pas d'élément élastique latéral (73), et comprend une partie ou la totalité de la partie creuse (70H).

4. Article portable jetable selon l'une des revendications 1 à 3,
dans lequel au moins un élément élastique latéral (73) est prévu sur le côté surface avant de la partie creuse (70H).

5. Article portable jetable selon l'une des revendications 1 à 4,
dans lequel les fronces montantes (60) s'élèvent à partir d'une position sur un côté central dans la direction de la largeur (WD) par rapport à un ou des éléments élastiques latéraux (73) et s'étendant le long de la direction avant-arrière (LD),
chaque fronce montante (60) comprend une feuille froncée (62) qui est pliée en deux au niveau d'une partie de pointe pour former une structure à deux couches, et un élément élastique de fronce allongé (63) est disposé le long d'au moins la couche intermédiaire de la structure à deux couches dans la direction avant-arrière (LD),
les deux parties latérales de chaque rabat latéral (70) comportent une première partie (P1) dans laquelle la structure à deux couches constituée de la feuille froncée (62) s'étend depuis un point de départ jusqu'au bord extérieur du rabat latéral (70) et une deuxième partie (P2) dans laquelle la structure à deux couches constituée de la feuille froncée (62) est repliée au niveau du bord extérieur du rabat latéral (70) et s'étend vers le centre de l'article portable jetable dans le sens de la largeur (WD), et
la double couche de feuille repliée est la première partie (P1) et la seconde partie (P2) de la feuille froncée (62).

6. Article portable jetable selon l'une des revendications 1 à 5,
dans lequel de multiples éléments élastiques latéraux (73) sont prévus à intervalles dans le sens de la largeur (WD), et
un taux d'étirement de l'élément élastique latéral (73) dans le la région latérale étirable (SG) dans un état déplié est plus bas car il est situé latéralement.

7. Article portable jetable selon l'une des revendications 1 à 6,
qui est un article portable jetable de type slip comprenant un corps extérieur (12F, 12B) formant au moins une partie inférieure du torse d'un corps avant (F) et un corps arrière (B), un corps intérieur (200) incorporant un absorbeur (56) et fixé au corps extérieur (12F, 12B) de manière à s'étendre du corps avant (F) au corps arrière (B), une ouverture au niveau de la taille (WO), et deux ouvertures pour les jambes gauche et droite (LO),
dans lequel les rabats latéraux (70) sont disposés sur les deux parties latérales du corps intérieur (200).
